# EUROPEAN PATENT SPECIFICATION

(11) **EP 2 195 455 B1**
(45) Date of publication and mention of the grant of the patent: **29.02.2012**
(21) Application number: 08802144.9
(22) Date of filing: 13.09.2008
(51) Int. Cl.: G01N 33/566

(54) **USE OF CLEC1B FOR THE DETERMINATION OF CARDIOVASCULAR AND THROMBOTIC RISK**
VERWENDUNG VON CLEC1B ZUR BESTIMMUNG DES HERZ-KREISLAUF- UND THROMBOSE-RISIKOS
UTILISATION DE CLEC1B POUR LA DÉTERMINATION DU RISQUE CARDIOVASCULAIRE ET DU RISQUE THROMBOTIQUE

(30) Priority: 24.09.2007 EP 07291134
(43) Date of publication of application: 16.06.2010
(60) Divisional application: 10163295.8
(73) Proprietor: SANOFI, 75013 Paris (FR)
(72) Inventor: KOZIAN, Detlef, 65926 Franfurt am Main (DE); WONEROW, Peter, 65926 Frankfurt am Main (DE); HERRMANN, Matthias, 65926 Frankfurt am Main (DE)
(74) Representative: Körner, Kathrin
(86) International application number: PCT/EP2008/007593
(87) International publication number: WO 2009/040002

(56) References cited:
- WO-A-2006/115295
- JP-A- 2007 070 359
- FULLER GEMMA L J ET AL: "The C-type lectin receptors CLEC-2 and Dectin-1, but not DC-SIGN, signal via a novel YXXL-dependent signaling cascade" JOURNAL OF BIOLOGICAL CHEMISTRY, vol. 282, no. 17, April 2007 (2007-04), pages 12397-12409, XP002470944 ISSN: 0021-9258
- DATABASE CA [Online] CHEMICAL ABSTRACTS SERVICE, COLUMBUS, OHIO, US; INOUE, KATSUE ET AL: "Novel mechanism of platelet aggregation: platelet membrane glycoprotein CLEC-2 and platelet signals" XP002470946 retrieved from STN Database accession no. 2007:477712
- SUZUKI-INOUE K ET AL: "A novel Syk-dependent mechanism of platelet activation by the C-type lectin receptor CLEC-2" BLOOD, W.B.SAUNDERS COMPANY, ORLANDO, FL, US, vol. 107, no. 2, 15 January 2006 (2006-01-15), pages 542-549, XP003003052 ISSN: 0006-4971

## Description

The invention relates to the use of single nucleotide polymorphisms (SNPs) and protein polymorphisms for identifying an increased risk of cardiovascular and/or thrombotic disorders and to primers and nucleic acids suitable for said use. In addition, the invention relates to the use of CLEC1B (C-TYPE LECTIN DOMAIN FAMILY 1, MEMBER B or alternatively C-TYPE LECTIN-LIKE RECEPTOR 2/CLEC-2, in the following referred to as CLEC1B) for finding active substances for preventing and treating cardiovascular and thrombotic disorders.

In the western world, cardiovascular and/or thrombotic disorders are among the leading causes of death among both sexes. Cardiovascular disorders comprise all disorders affecting the function of the heart and include especially disorders of the cardiac tissue and the cardiac vessels. Thrombotic disorders comprise all disorders affecting pathological states of the blood flow either associated with reduced blood flow as a consequence of vessel blockage of associated with increased bleeding tendency.

Coronary heart disease, in particular coronary artery disease, can be regarded as one of the major causes of cardiovascular disorders. Angina, also called angina pectoris, is temporary chest pain or a sensation of pressure that occurs when the heart muscle is not supplied with enough oxygen. When the coronary arteries are narrowed or blocked so that blood flow to the heart muscle cannot increase to meet the increased demand for oxygen, ischemia may occur as a result thereof, causing said pain (i.e. said angina pectoris). Normally, angina pectoris results from coronary artery disease but may also be caused by other coronary heart diseases. Not every ischemia of the heart muscle causes the pain or sensations of pressure connected with angina pectoris. Ischemia of the heart muscle of this kind, i.e. without angina pectoris, is referred to as silent ischemia. The danger of silent ischemia lies in the fact that the damage to the heart muscle is not noticed by the individual affected. Therefore, the patient or the physician in charge is often unable to recognize possible damage to the heart tissue, until said damage ultimately results in a myocardial infarction. For this reason, there is a great demand for diagnostic methods and means for recognizing vascular and cardiac degenerations, which enable a diagnosis, and thus a therapeutic intervention, as early as possible.

Due to the high socio-economic and personal burdens associated with cardiovascular and/or thrombotic disorders, there is a great need for early diagnosis and treatment of said disorders.

It is thus the object of the present invention to provide improved methods for the diagnosis and treatment of cardiovascular and/or thrombotic disorders.

According to the invention, this object is achieved by using single nucleotide polymorphisms (SNPs) or in the CLEC1B gene or polymorphisms of the CLEC1B protein for the identification of cardiovascular and/or thrombotic disorders in a biological sample taken from an individual to be examined, wherein one or more of the SNPs at position 251 of the CLEC1B gene (according to SEQ ID NO 1) is analysed.

This can e.g. be achieved by analyzing a CLEC1B nucleic acid (i.e. RNA or DNA (such as, for example, cDNA or genomic DNA)) for the presence of the single nucleotide polymorphism thymidine (T) → cytidine (C) at position 251 of the CLEC1 B nucleic acid sequence according to reference sequence NM_016509 (or the corresponding position in the CLEC1B genomic sequence (e.g. according to SEQ ID NO NT_009714)) and/or by analyzing a CLEC1 B protein for the presence of the protein polymorphisms serine (Ser) → proline (Pro) at position 24 of the CLEC1 B protein according to reference sequence NP_057593 or by analyzing the amount or functional properties of protein or mRNA of CLEC1 B present in the sample taken.

The type of the nucleotide or amino acid at any relevant position within the CLEC1B nucleic acid or protein can be determined here on the basis of common methods. By knowing the type of the nucleotide or amino acid at certain positions, the skilled worker can readily determine the cardiovascular and/or thrombotic risk group to which the individual belongs from whom the biological sample was derived.

CLEC1 B belongs to the C-type lectin superfamily of transmembrane proteins (Kanazawa et al., 2007). CLEC1B has been cloned in 2000 and maps to human chromosome 12 within a NK gene complex. CLEC1B consists of 229 amino acids and has an apparent molecule mass of 27 kDa (Colonna et al., 2000; Sobanov et al., 2001). CLEC1B is expressed in monocytes, granulocytes and dendritic cells (Kanazawa et al., 2007). Recently the expression of CLEC1B on platelets has been described (Suzuki-Inoue et al., 2006). CLEC1B has been identified as a novel binding protein of rhodocytin suggesting that the receptor is involved in platelet activation by the snake venom toxin. The recently solved crystal structure of CLEC1B further supports the concept of rhodocytin being a ligand for this receptor (Watson et al., 2007). Besides rhodocytin also antibodies against CLEC1B are able to initiate the phosphorylation of tyrosine kinases in platelets, indicating that the receptor is able mediate platelet activation upon agonist engagement (Suzuki-Inoue et al., 2006; Fuller et al., 2007). Although the exact function of CLEC1B on platelet is still not elucidated, the receptor seems to be involved in the dissemination of the human immunodeficiency virus type 1 (HIV-1) in infected patients. It seems that platelets are getting occupied by HIV-1 via the attachment factors CLEC1B and lectin dendritic cell-specific intracellular adhesion molecule 3-grabbing nonintegrin (DC-SIGN), both being expressed on thrombocytes (Chaipan et al., 2006).

The sequence of the CLEC1B gene is known in the art. The CLEC1B gene is located on chromosome 12p13.2. The coding nucleic acid sequence of said gene can be retrieved under the number NM_016509 at the NCBI database. A contiguous genomic sequence of the CLEC1B gene can be gained from the Homo sapiens chromosome 12 genomic contig NT_009714 at the NCBI database. The derived protein sequence can be retrieved under the number NP_057593 at the NCBI Nucleotide Database. NCBI is the National Center for Biotechnology Information (postal address: National Center for Biotechnology Information, National Library of Medicine, Bethesda, MD 20894, USA; web address: hftp://www.ncbi.nim.nih.gov/).

The present invention relates to studies of the CLEC1B gene at the chromosomal level in a clinical cohort of patients, carried out by the inventors to estimate the influence of variations in the CLEC1B gene and/or protein on the clinical or pathophysiological phenotype of a carrier of such variants.

Single nucleotide polymorphisms (SNPs) are variants of a particular nucleotide sequence containing substitutions at individual positions and are well known to the skilled worker. The term protein polymorphism as used herein comprises any change of the protein primary (i.e. amino acid sequence), secondary (i.e. protein folding) and/or tertiary structure (i.e. the assembly of a protein from different polypeptide subunits) and preferably comprises changes caused by one or more SNPs of the gene encoding a protein; examples are, e.g. amino acid exchanges, amino acid deletions or truncations of the protein.

Different single nucleotide polymorphisms (SNPs) of the CLEC1B gene are known in the prior art and publicly accessible at the Ensembl database, e.g. at hftp://www.ensembl.org/Homo sapiens/genesnpview?db=core:gene=ENSG00000165 682. Also, the above-identified SNP at position 251 of the CLEC1 B nucleic acid sequence according to reference sequence NM_016509 (or at the corresponding position in the CLEC1 B genomic sequence, e.g. at position 201 with respect to SEQ ID NO:3) is known (refSNP ID: rs2273986), and can be retrieved from the NCBI database under accession number rs_2273986 and can especially be retrieved by use of the following link:
htto://www.ncbi.nlm.nih.gov/SNP/snp ref.cqi?rs=2273986

This link also discloses part of the genomic sequence surrounding the SNP according to present invention (see also SEQ ID NO: 3).

However, the association of said polymorphism of CLEC1B with an association for having or experiencing cardiovascular and/or thrombotic disorders has not been known or described, so far and is completely surprising.

Experiments of the inventors have demonstrated for the first time that a certain variation in the CLEC1B gene occurs with statistically significant frequency in humans suffering from cardiovascular and/or thrombotic disorders. To assess a possible relation of SNPs within or variants of the CLEC1 B gene or protein with the onset and the development of diseases, the genetic variant Serine-Proline at position 24 of the CLEC1 B protein has been analyzed in detail and genotype-phenotype association analyses have been performed in a well-defined patient cohort. The result is the 5 surprising finding that said CLEC1B polymorphism correlates with a cardiovascular and/or thrombotic risk or disorders. The correlation of polymorphisms of theCLEC1 B gene with a predisposition for this type of disease has never been described before and, in view of the data published on CLEC1B, so far, is to be regarded as completely surprising.

The different aspects of present invention are applicable for all animals or human beings. A preferred embodiment concerns the application for mammals and/or humans. Accordingly, the term CLEC1B (with respect to nucleic acid, protein, polymorphisms etc.) refers to CLEC1 B from any animal species or Homo sapiens. Preferred embodiments encompass CLEC1 B from mammals and/or Homo sapiens (hs) CLEC1 B.

Thus, another aspect of present invention concerns the use of a CLEC1 B protein or nucleic acid or of a functional fragment thereof for the identification of cardiovascular and/or thrombotic risk or disorders in a biological sample taken from an individual to be examined, by analyzing a CLEC1 B nucleic acid for the presence of the single nucleotide polymorphism thymidine (T) → cytidine (C) at position 251 of the CLEC1B nucleic acid sequence according to reference sequence SEQ ID No 1 and/or by analyzing a CLEC1B protein for the presence of the protein polymorphisms serine (Ser) → proline (Pro) at position 24 of the CLEC1 B protein according to reference sequence SEQ ID No 2.

In the following, the most frequently occurring nucleotide or amino acid occurring at a given position within the CLEC1B gene and/or nucleic acid or protein is referred to as the most frequent variant or the "wild type".

CLEC1 B -T251T describes the group of individuals who have a thymidine (T) at position 251 with respect to the reference sequence NM_01 6509 (or at the corresponding position in the CLEC1 B genomic sequence) on both alleles of the CLEC1 B gene. This polymorphism leads to a CLEC1 B protein having the amino acid serine (Ser or S) at position 24 (Ser24) with respect to the reference sequence NP_057593. Said persons are homozygous with respect to said CLEC1 B variant. As can be gained from Table 2, the nucleotide T is the most frequent variant at position 250 of the CLEC 1 B gene and the amino acid serine the most frequent variant at position 24 of the CLEC1 B protein.

CLEC1B-T251C describes the group of individuals who have a thymidine (T) at position 251 with respect to the reference sequence NM_016509 (or at the corresponding position in the CLEC1B genomic sequence) on one allele of the CLEC1B gene and a cytidine (C) at position 250 with respect to the reference sequence NM_016509 (or at the corresponding position in the CLEC1B genomic sequence) on the other allele of the CLEC1 B gene. This polymorphism leads to a CLEC1B protein having the amino acid serine (Ser or S) at position 24 (Ser24) with respect to the reference sequence NP_057593 and a CLEC1 B protein having the amino acid proline (Pro or P) at position 24 (Pro24) with respect to the reference sequence NP_057593. Said persons are heterozygous with respect to said CLEC1 B variant.

CLEC1B -C251C describes the group of individuals who have a cytidine (C) at position 251 with respect to the reference sequence NM_016509 (or at the corresponding position in the CLEC1B genomic sequence) on both alleles of the CLEC1 B gene. This polymorphism leads to a CLEC1 B protein having the amino acid proline (Pro or P) at position 24 (Pro24) with respect to the reference sequence NP_057593. Said persons are homozygous with respect to said CLEC1 B variant.

Genetic variations in the CLEC1B gene may be detected, for example:
a) by direct detection of genetic variations at the chromosomal DNA level by way of molecular-biological analysis of the CLEC1B gene which may contain said genetic variations, here in particular the region around position 251 according to the reference sequence NM_016509 of the CLEC1 B coding sequence (or the corresponding position in the CLEC1B genomic sequence),
b) via detection by measuring CLEC1 B mRNA expression,
c) by detection of protein polymorphisms within the CLEC1B protein, here in particular at positions 24 of the CLEC1 B polypeptide chain, according to reference sequence NP_057593, and
d) by indirect detection by way of determining the amounts and/or activity of CLEC1B protein present in cells, tissues or body fluids by means of protein-chemical methods.

Genetic variations or polymorphisms at the nucleic acid level (here chromosomal DNA) in the CLEC1 B gene the position in the above reference sequences may be detected, for example, by
1) Methods based on the sequencing of the nucleic acid sequence of said region of the CLEC1B gene (e.g. pyrosequencing, sequencing using radio labeled or fluorescent dye-labeled nucleotides or via mass spectrometric analysis of said nucleic acid sequence);
2) Methods based on hybridization of nucleic acid sequences of said region of the CLEC1B gene (e.g. by means of "DNA micro arrays");
3) Methods based on the analysis of amplification products of the nucleic acid sequence of said region of the CLEC1B gene (e.g. TaqMan analyses).

Genetic variations or polymorphisms at the nucleic acid level (here chromosomal) DNA) in the CLEC1 B gene the above position with respect to the above reference sequences may also be detected, for example, on the basis of measuring expressed CLEC1B mRNA via
1) Methods based on the hybridization of nucleic acid sequences of the CLEC1B gene (e.g. by means of "DNA micro arrays", Northern blot analyses);
2) Methods based on the analysis of amplification products of the nucleic acid sequence of the CLEC1B gene (e.g. "TaqMan" analyses, differential RNA display, representational difference analysis).

The protein polymorphisms within the CLEC1 B protein at one or both of the positions 24 with respect to the protein sequence according to the reference sequence NP_057593 can e.g. be detected by means of specific antibodies being able to discriminate between e.g. a serine or a proline at position 24 within the CLEC1B protein, or by alternative biochemical or molecular biological methods suited for the discrimination between CLEC1 B variants with either proline or serine at position 24 of the CLEC1B protein sequence according to reference sequence NP_057593.

In addition, genetic variations or polymorphisms at the above position within one of the above reference sequences may be detected via analyzing the amount and/or activity of the CLEC1B protein. The amount and/or activity of the CLEC1 B protein may be detected, for example, on the basis of
1) Methods based on quantitative detection of the amount of the CLEC1B protein (e.g. Western blot analyses, ELISA test), or
2) Methods based on functional detection of the activity of the CLEC1B protein via in vitro test systems, for example in human cells, animal cells, bacteria and/or yeast cells.

The detection of the genetic variations or polymorphisms in the CLEC1B gene at one of the above positions may be used, for example, as (a) genetic marker for evaluating the risk of cardiovascular and/or thrombotic disorders, e.g.: peripheral vascular disease, high blood pressure, stroke/PRIND/TIA. instable angina, premature myocardial infarction, myocardial infarction, and/or coronary heart diseases (b) marker for preventative treatment of cardiovascular and/or thrombotic disorders, e.g.: peripheral vascular diseases, high blood pressure, stroke/PRIND/TIA, instable angina, premature myocardial infarction, myocardial infarction and/or coronary heart diseases in carriers of the corresponding genetic variants, (c) marker for adapting the dosage to be administered of a pharmaceutically active substance for cardiovascular and/or thrombotic disorders, e.g.: peripheral vascular diseases, high blood pressure, stroke/PRIND/TIA, instable angina, premature myocardial infarction, myocardial infarction and/or coronary heart diseases, (d) marker for determining the high throughput screening strategy for identifying a pharmaceutically active substance for cardiovascular and/or thrombotic disorders, e.g.: peripheral vascular diseases, high blood pressure, stroke/PRIND/TIA, instable angina, premature myocardial infarction, myocardial infarction and/or coronary heart diseases (e) marker for identifying the relevant individuals or patients for clinical studies in order to test the compatibility, safety and efficacy or a pharmaceutical substance for cardiovascular and/or thrombotic disorders, e.g.: peripheral vascular diseases, high blood pressure, stroke/PRIND/TIA, instable angina, premature myocardial infarction, myocardial infarction and/or coronary heart diseases, and (f) basis for developing test systems for analyzing the genetic variation in the CLEC1B gene at the DNA, RNA or protein level.

Thus, another aspect of present invention concerns the use of a CLEC1 B protein or nucleic acid or of a fragment thereof for the identification of increased risk for developing cardiovascular and/or thrombotic disorders in a biological sample taken from an individual to be examined.

Yet another aspect of present invention concerns a method for identifying cardiovascular and/or thrombotic disorders or an increased risk for developing cardiovascular and/or thrombotic disorders in an individual, which comprises examining a sample taken from an individual for the type of nucleotide, which is present at position 251 on one or both alleles of the CLEC1 B gene according to reference sequence NM_016509 (or at the corresponding position in the CLEC1 B genomic sequence), the type of nucleotide present at said position being indicative of the risk of said individual to suffer from or develop cardiovascular and/or thrombotic disorders, the presence of a cytidine nucleotide at said position being indicative for an increased risk of said individual to suffer from or develop cardiovascular and/or thrombotic disorders, the presence of a nucleotide other than cytidine, preferably a thymidine indicating a lowered risk of said individual to suffer from or develop cardiovascular and/or thrombotic disorders.

Present invention thus also relates to a method for identifying cardiovascular and/or thrombotic disorders or an increased risk for developing cardiovascular and/or thrombotic disorders in an individual, which comprises examining a sample taken from an individual for the type of amino acid present at position 24 of the polypeptide chain of CLEC1 B protein, the type of amino acid present at said one or more positions being indicative of the risk of said individual to suffer from or develop cardiovascular and/or thrombotic disorders.

According to one embodiment, the invention concerns a method for identifying cardiovascular and/or thrombotic disorders or an increased risk for developing cardiovascular and/or thrombotic disorders in an individual, which comprises examining a sample taken from the individual, as to whether the amount of CLEC1 B mRNA and/or amount or functional activity of protein present in said sample is different from that of one or more reference samples. The presence of a different amount indicating an increased risk of said individual to suffer from or to develop cardiovascular and/or thrombotic disorders.

The change in the amount of CLEC1B, i.e. the change in CLEC1 B levels, may be caused here by influencing all levels of expression (transcription, translation, splicing), post-translational modification, transport of the protein or proprotein, or influence on protein stability as well as by influences due to signal transduction pathways acting on CLEC1B expression.

A reference sample can e.g. be a sample taken from one or more individuals having the following genomic and/ or protein variants: a nucleotide other than thymidine, and preferably a cytidine, at position 251 of the CLEC1B nucleotide sequence according to reference sequence NM_016509 (or at the corresponding position in the CLEC1 B genomic sequence) on one or both alleles of the CLEC1B gene.

Yet another aspect of present invention concerns a method of determining the risk of suffering from cardiovascular and/or thrombotic disorders comprising analyzing an isolated sample of an individual for the presence of the before-mentioned SNP or protein polymorphism and calculating the estimated risk on basis of the age of the patient and the type of nucleotide or amino acid present at the positions 251 of the CLEC1B coding sequence or at position 24 of the CLEC1B protein. A basis for the risk determination is the results according to Tables 3-5.

The cardiovascular and/or thrombotic disorder in any of the different embodiments and aspects of present invention can e.g. be peripheral vascular disease, high blood pressure, stroke/PRIND/TIA, instable angina, myocardial infarction, early myocardial infarction, coronary artery disease, coronary heart disease and any pathological state that necessitates the undergoing of a coronary angioplasty.

The nucleotide position indicated herein refers to the position of the nucleotide in the reference sequence NM_016509 (or at the corresponding position in the CLEC1B genomic sequence).

With respect to the CLEC1B amino acid sequence the amino acid position refers to the reference sequence NP_057593.

The positions start from 1 for the first amino acid or nucleotide of the reference sequence unless, for nucleotide sequences, the number of the nucleotide is preceded by a + or a -, in which case the nucleotide position is given with respect to the site of translation.

Standard abbreviations will be used herein below synonymously for nucleotides and amino acids (i.e. three- or one-letter code).

A nucleic acid can be any oligo- or polynucleotide, the term "oligonucleotide" concerning nucleic acids of 2 to 25 nucleotides and the term "polynucleotide" referring to nucleic acids having 26 and more nucleotides.

In the present application, the terms protein sequence, amino acid sequence and polypeptide sequence can be used synonymously.

So far, no data connecting clinical effects with CLEC1B variants in humans have been disclosed. Surprisingly, the studies by the inventors have been able to closely connect the presence of a CLEC1 B variant at position 24 of the CLEC1B protein and especially the variant Ser→Pro at position 24 of the CLEC1B protein with a predisposition for cardiovascular and/or thrombotic disorders.

The detection of genetic polymorphisms of the CLEC1B gene, in particular the nucleotide exchange T→C at position 251 according to reference sequence NM_016509 (or at the corresponding position in the CLEC1B genomic sequence) may serve, for example, as genetic marker for preventive treatments and preventive measures (medication, lifestyle), (a) in order to delay or even to prevent the onset of cardiovascular and/or thrombotic disorders, such as peripheral vascular disease, high blood pressure, stroke/PRIND/TIA (PRIND stands for prolonged ischemic attack; TIA stands for transitory ischemic attack), instable angina, myocardial infarction, early myocardial infarction, coronary heart disease and any pathological state that necessitates the undergoing of a coronary angioplasty, or to alleviate or stop the severity of the later course and the pathological sequelae, or (b) as genetic marker for adjusting a pharmaceutical dosage or (c) as genetic marker for designing a screening for pharmaceuticals or (d) as genetic marker for identifying and, where appropriate, selecting patients in particular treatments or medical studies.

The methods of the invention enable a predisposition for cardiovascular and/or thrombotic disorders to be identified early, thereby making possible the early use of preventive or curative treatment measures, before classical symptoms such as sensations of pain as a result of tissue damage occur: identification of the polymorphism of the invention or of a changed steady state level or function of CLEC1 B mRNA or protein by the skilled worker in charge, gives a clear indication for the treating or examining physician to screen for an already persisting damage to vessels or heart tissue, or to administer preventive pharmaceuticals, or to suggest a change in lifestyle even before corresponding damage or pain occurs.

In addition, the novel finding of a connection between said variants and the predisposition for cardiovascular and/or thrombotic disorders allows the use of more effective treatments by hinting at a change in the dosage of particular pharmaceuticals or at the necessity of changing the treatment of patients with said polymorphism in the CLEC1B coding sequence or protein.

Accordingly, the present invention also relates to the use of
a) one or more single nucleotide polymorphisms (SNPs) in the CLEC1 B gene,
b) one or more protein polymorphisms in the CLEC1B protein and/or
c) an CLEC1 B protein or nucleic acid or of a functional fragment thereof for adapting the dosage of a pharmaceutical for the prevention and/or treatment of cardiovascular and/or thrombotic disorders.

Moreover, present invention relates to a method for adapting the dosage of a pharmaceutical for the prevention and/or treatment of cardiovascular and/or thrombotic disorders in an individual, which method comprises examining a taken sample of the individual for
a) the type of the nucleotide, which is present at positions 251 on either on one or both alleles of the CLEC1B gene according to reference sequence NM_016509 (or at the corresponding position in the CLEC1B genomic sequence) the presence of a cytidine nucleotide at said position being indicative for an increased risk of said individual to suffer from or develop cardiovascular and/or thrombotic disorders, the presence of a nucleotide other than cytidine, preferably a thymidine indicating a lowered risk of said individual to suffer from or develop cardiovascular and/or thrombotic disorders; and/or
b) the type of amino acid present at position 24 in the CLEC1B protein according to reference sequence NP_057593, the presence of a proline at said position being indicative of an increased risk of said individual to suffer from or develop cardiovascular and/or thrombotic disorders, the presence of an amino acid other than proline, preferably a serine at position 24 of the CLEC1 B protein (according to SEQ ID NO 2) indicating a lowered risk of said individual to suffer from or develop cardiovascular and/or thrombotic disorders,
said dosage being adapted dependent of the type of nucleotide or amino acid present at said positions.

One embodiment comprises examining the sample taken from the individual as to whether either one or both alleles of the CLEC1B gene have the following SNP: a thymidine at position 251 of the CLEC1B coding sequence according to reference sequence NM_016509 (or at the corresponding position in the CLEC1 B genomic sequence), the dosage of the pharmaceutical being decreased or increased in the presence of the polymorphism.

Another embodiment comprises examining the sample taken from the individual as to whether either or both alleles of the CLEC1 B gene have a nucleotide other than the listed above at the above-listed position, the dosage of the pharmaceutical being decreased or increased in the presence of another nucleotide. The other nucleotide is preferably a cytidine at position 251 of the CLEC1 B sequence according to reference sequence NM_016509 (or at the corresponding position in the CLEC1B genomic sequence).

According to another embodiment of present invention, the method for adapting the dosage of a pharmaceutical for the treatment and/or prevention of cardiovascular and/or thrombotic disorders of an individual comprises examining a sample taken from the individual, as to whether the amount of CLEC1B mRNA and/or protein present in said sample is different from that of one or more reference samples. A reference sample can e.g. be a sample taken from an individual having one or more of the following genomic and/or protein variants: a nucleotide other than thymidine, and preferably a cytidine, at position 251 of the CLEC1B sequence NM_016509 (or at the corresponding position in the CLEC1B genomic sequence) on one or both alleles of the CLEC1 B gene, said dosage being adapted depending on whether the amount of protein and/or mRNA in the taken sample of the individual is different from that of the reference sample or reference samples from one or more individuals having one or more of the variants.

The presence of a CLEC1B gene variant, in particular that of the CLEC1 B-T251C variant or the CLEC1B-C251C variant, has indicator function. The prior art knows a multiplicity of pharmaceuticals for treating or preventing cardiovascular and/or thrombotic disorders. Since not all pharmaceuticals have the same effect on all patients with the same disease, patients which are treated with cardiovascular pharmaceuticals for the first time normally have to be "adjusted" to the latter, i.e. the treating physician de facto has to test on the individual patient as to which dosage of which pharmaceutical has the desired effect with side effects as small as possible. The disadvantage here is the fact that it is not known beforehand, whether the symptoms in the patient are alleviated or stopped by the pharmaceutical administered (at the given dosage). It is also not possible beforehand to assess accurately, whether said patient will suffer from an undesired side effect.

In this context, identifying the patients as patients having a certain CLEC1B gene variant or having an amount of CLEC1B nucleic acid or protein associated with a certain probability of suffering from cardiovascular and/or thrombotic disorders prior to the treatment, may improve the predictability of the success of treatment with a particular pharmaceutical: the connection of particular variants of the CLEC1B gene with the occurrence of cardiovascular and/or thrombotic disorders suggests that CLEC1 B variations of this kind concur with physiological changes in the individual which ultimately have the effect that said individuals have a higher or lower probability of suffering from a cardiovascular and/or thrombotic disorders than other individuals. The different efficacy of each pharmaceutical in different individuals must be seen against such a background of different physiological provision of the individual patients. Assigning an individual to such a group of patients with a particular physiological background would allow particular pharmaceuticals which have been proven in clinical studies to be particularly active here to be preferably used and pharmaceuticals which are less active or more likely linked to undesired side effects in this group of patients, compared to patients without said variant, not to be used from the outset.

Normally, a classification of this kind of individual patients prior to a treatment is not possible. Only the knowledge of the connection between the polymorphism on which the invention is based with the occurrence of cardiovascular and/or thrombotic disorders makes this possible. Thus, pharmaceuticals which have shown good success in the treatment of patient groups having the same gene variant in clinical studies may preferably be used on patients having a variation in the CLEC1B gene, whereas pharmaceuticals which are less effective in said patient group or which have a higher probability of undesired side effects than in patient groups having a different gene variant would not be used from the outset. This would reduce the risk for the patient who is "adjuster to a pharmaceutical and increase the probability of a successful treatment.

Accordingly, a further aspect of present invention relates to the use of
a) one or more single nucleotide polymorphisms (SNP) in the CLEC1B gene,
b) one or more polymorphisms in the CLEC1B protein and/or
c) an CLEC1B protein or nucleic acid or of a fragment thereof
for identifying individuals responding to a pharmaceutical for the treatment and/or prevention of cardiovascular and/or thrombotic disorders.

Such identification may be carried out, for example, by examining a sample taken from an individual as to whether (a) either or both alleles of the CLEC1 B gene have the following variant, the presence of said nucleotide being an indicator for the individual from whom the sample has been derived responding to the pharmaceutical: a thymidine at position 251 of the CLEC1 B sequence NM_016509 (or at the corresponding position in the CLEC1B genomic sequence);
(b) either one or both alleles of the CLEC18 gene have one or more of the following variants, the presence of which is an indicator for the individual from whom the sample has been derived responding to the pharmaceutical: a nucleotide other than thymidine, and preferably a cytidine at position 251 of the CLEC1B sequence NM_016509 (or at the corresponding position in the CLEC1B genomic sequence); (c) the amount of CLEC1 B mRNA and/or protein in the sample is different from that in one or more comparative/reference samples, e.g. from one or more reference individuals having a known genetic background with respect to the CLEC1B gene (e.g. the polymorphism according to (a) or (b)), the presence of a different amount being an indicator for the individual from whom the sample has been derived responding to the pharmaceutical or (d) the CLEC1 B protein has the polymorphism Ser→Pro at position 24 of CLEC1B protein reference sequence NP_057593, with other methods and procedures for identification also being conceivable.

The determination of the type of nucleotide for the different aspects of present invention can be performed according to methods known in the art. This can e.g. be achieved by
a) providing an isolated biological sample comprising genomic DNA or providing isolated genomic DNA;
b) amplifying a nucleic acid by carrying out a PCR reaction using primers able to amplify a nucleic acid comprising the positions 251 of the CLEC1 B sequence NM_016509 (or at the corresponding position in the CLEC1B genomic sequence);
c) Sequencing the nucleic acid.

Another possibility to determine the type of nucleotide is e.g. by
a) providing an isolated biological sample comprising genomic DNA or providing isolated genomic DNA;
b) Immobilizing the genomic DNA on a suitable support;
c) Hybridizing to the immobilized DNA one or more probes, which, under standard conditions, are capable of binding specifically to nucleic acids having a (genomic) CLEC1 B sequence and which have a specificity for a particular nucleotide at position 251 of the CLEC1 B sequence according to reference sequence NM_016509 (or at the corresponding position in the CLEC18 genomic sequence).

According to another possibility the type of nucleotide can also be determined on the basis of the above two methods, but using cDNA generated from mRNA instead of using genomic DNA.

The amount of mRNA can e.g. be determined by
a. Providing a biological sample comprising mRNA or providing isolated mRNA from the sample of a);
b. Amplifying a nucleic acid by RT-PCR using primers having the ability to amplify a nucleic acid derived from the CLEC1 B mRNA;
c. Quantifying the amount of the amplified nucleic acid and comparing it with the amount of nucleic acid amplified in at least one reference sample (i.e. positive and/or negative control samples).

The concept of positive or negative controls for verifying the result of any given analytical (biological, biochemical or chemical) reaction is well known to the skilled artisan. It comprises e.g. reactions performed in the same manner as the original analytical experiment, but lacking one or more defined components (e.g. lacking CLEC1 B protein or mRNA or lacking a specific CLEC1B antibody, etc.) to discriminate specific signals which are the outcome of said experiments from so called "background" signals (artificial signals created by a given analytical method) (negative controls). It also comprises reactions performed in the same manner as the original analytical experiment but using an additional component that will result in a known signal for verifying that the reaction conditions in general work (positive control).

Another possibility of determining the amount of mRNA is e.g. by means of
a. Providing a biological sample, which comprises mRNA or providing isolated mRNA;
b. Transferring the mRNA to a suitable support;
c. Detecting and quantifying the CLEC1B mRNA on the support by means of at least one suitable probe;
d. Comparing with the amount of CLEC1B mRNA from one or more reference samples (e.g. positive and/or negative control samples).

Yet another possibility is a method comprising:
a. Providing a histological sample of the individual;
b. Detecting the amount of CLEC1B mRNA by way of hybridization reaction with a suitable mRNA probe, detecting and quantifying the hybridized probe;
c. Comparing the amount of CLEC1 B mRNA with that in one or more reference samples (e.g. positive and/or negative control samples).

The determination of the amount of protein or the identification of a protein polymorphism can e.g. be achieved by
a. Providing a biological sample of the individual to be examined, which comprises protein;
b. Preferably isolating the protein from the sample of a.;
c. Transferring the protein to a suitable support;
d. Detecting the protein by means of at least one antibody specific CLEC1B protein or specific for a certain CLEC1B protein polymorphism; and
e. Quantifying the signal and comparing it with the signal obtained from at least one reference sample (i.e. a negative control and/or a positive control sample).

Another possibility of determining the amount of protein or identifying a certain protein polymorphism is a method comprising:
a. Providing a histological sample of the individual;
b. Detecting the amount of CLEC1B protein by way of a binding reaction with a suitable CLEC1B antibody, detecting and quantifying said amount;
c. Comparing the amount of CLEC1 B protein with that in one or more reference samples (e.g. positive and/or negative control samples).

The determination of certain protein polymorphisms can e.g. be achieved by using an antibody against CLEC1 B protein having detectably higher binding affinity for a CLEC1 B protein with a serine at position 24 of the polypeptide chain than for a CLEC1 B protein with another amino acid, especially with a proline at position 24 of the polypeptide chain (position of amino acid according to reference sequence NP_057593).

Furthermore, the determination of certain protein polymorphisms can be achieved by using any suitable alternative molecular biology or biochemical method known in the art, like mass spectrometry, suited for differentially detecting a CLEC1 B protein with a serine at position 24 of the polypeptide chain and a CLEC1 B protein with another amino acid, especially with a proline at position 24 of the polypeptide chain.

In this connection, the term sample or taken or isolated sample refers to biological material taken from the patient. Biological material may include, inter alia: the cells or preparations or parts of a tissue or an organ or body fluids (e.g. lymph, saliva, blood, skin, connective tissue), or cells, preferably cells which are easy to remove, such as, for example, mucosal cells. Biological material of this kind may be obtained by common techniques such as taking a swab, taking a blood sample, tissue puncture or surgical techniques (e.g. biopsies). The samples are preferably histological specimens, cell preparations, cells, for example mucosal cells, cellular tissue, purified DNA, mRNA or protein or a body fluid such as saliva, lymph or blood or extracts or preparations of said samples thereof. The purification of naturally occurring molecules from cells or tissues and the preparation of cell or tissue extracts are well known to the skilled person (see also examples of the standard literature listed below). DNA/RNA or protein preparations can be obtained there from by means of common techniques.

Since CLEC1 B has been identified in the present application for the first time as being connected to cardiovascular and/or thrombotic disorders, an example concerns the use of a CLEC1 B protein or nucleic acid or of a functional fragment thereof for finding active substances for treating and/or preventing cardiovascular and/or thrombotic disorders.

According to one embodiment of the different aspects of present invention, CLEC1B, the derivative or fragment thereof can be used as an isolated molecule.

In the context of this invention, the term "isolated molecule", especially with respect to CLEC1 B, refers to CLEC1 B nucleic acids or polypeptides or fragments thereof purified from natural sources (i.e. removed from their natural environment) as well as purified recombinant molecules (wherein the term purified comprises a partial purification as well as a complete purification). The isolation of nucleic acids is well known in the art (see also literature on standard laboratory procedures below).

The use according to present invention allows for the identification of novel substances for the prevention and/or treatment of cardiovascular and/or thrombotic disorders. The use according to present invention comprises the identification of substances with the desired characteristics as well as the further characterisation of substances already identified to be useful for the prevention and/or treatment of cardiovascular and/or thrombotic disorders.

A substance as to be employed for the different aspects of present invention can be any biological or chemical substance or natural product extract either purified, partially purified, synthesized or manufactured by means of biochemical or molecular biological methods.

A substance considered as being active in preventing or treating cardiovascular and/or thrombotic disorders in the sense of the different aspects of present invention can be any substance having an influence of one of the functions of CLEC1B or on the expression, amount or steady state level of CLEC1 B mRNA or protein in a biological system.

To this end, the substance can modulate any of the functions of CLEC1B (e.g. those as defined above or herein below). CLEC1 B protein activity can be modulated by the substance e.g. by direct interaction and interference with the function of CLEC1 B polypeptide/protein or fragments thereof. The substance can also modulate the expression of CLEC1B. e.g. on the level of transcription (initiation, elongation, termination), of transcript- or translate-processing (especially post-translational processing of the prepro- or pro-protein into the active form, which may also comprise C-terminal truncation of the full-length protein lacking the propeptide domain), transcript- or translation product stability or translation. Moreover it can modulate the posttranslational processing, modification, protein folding etc. of CLEC1B. The substance can exert the above effects directly or indirectly (indirectly meaning i.e. by interfering (positively or negatively) with natural signalling cascades having influence on CLEC1 B function / protein activity / expression etc.). Moreover the substance can also mimic CLEC1B activity (i.e. take over its function/role).

A fragment of CLEC1 B can be any polypeptide or nucleic acid that is shorter than the corresponding wild type, e.g. shorter than Homo sapiens (hs) CLEC1B or the polypeptide. A functional fragment of CLEC1B is any fragment (either polypeptide or nucleic acid), which exhibits at least one of the functions of CLEC1B.

A derivative of CLEC1B or of a CLEC1B fragment can be any modification of a CLEC B nucleic acid, polypeptide or of a fragment thereof. Derivatives comprise, e.g. modifications of the amino acid or nucleotide sequence or any other kind of modification, such as a chemical or biological modification e.g. leading to the stabilization of the polypeptide or nucleic acid (such as phosphoorothioate modifications or other kinds of modifications of the nucleic acid backbone or of exchanges of the bonds between amino acids, etc.), or enabling a specific targeting of the polypeptide or nucleic acid to certain cells or facilitating its entry into or uptake by cells (such as cell-permeant phosphopeptides, ortho coupling to cell-permeant peptide vectors, e.g. based on the antennapedia/penetratin, TAT, and signal-peptide based sequences; or coupling to parts of ligands for specific transporters or importers).

The term "functional derivative" of CLEC 1 B comprises any kind of modification of CLEC18 with respect to the naturally occurring form (either polypeptide or nucleic acid), which at least has one of the functions of CLEC1B. Present invention also comprises functional derivatives of fragments of CLEC1 B.

With regard to CLEC1 B nucleic acids or fragments thereof, CLEC1 B function comprises for example the ability to interact with other molecules, such as, for example, specific hybridization primers or probes, the ability to control transcription of a downstream coding sequence, to code for CLEC1B protein, etc.). Functions of CLEC1 B comprise also the ability of CLEC18 (protein or nucleic acid) or fragments thereof to interact with other molecules (comprising, but not limited to, proteins or protein fragments, nucleic acids (i.e. CLEC1 B nucleic acids to specifically hybridise with other nucleic acids), synthetic molecules (i.e. CLEC1B protein or fragments to specifically interact with synthetic drugs)).

The identification of active substances can e.g. be performed by means of one or more of the methods identified herein below, such as:
A method for identifying substances active in preventing or treating cardiovascular and/or thrombotic disorders comprising:
   a. Contacting a CLEC1B protein or functional fragment or derivative thereof with a test substance; and
   b. Determining whether the test substance modulates the activity of the CLEC1B protein or functional fragment or derivative thereof.

A method for identifying substances active in preventing or treating cardiovascular and/or thrombotic disorders comprising:
a. Contacting a cell, which has a detectable amount or activity of CLEC1 B or of a functional fragment or derivative thereof, with a test substance;
b. Determining whether the test substance is able to modulate the amount or activity of CLEC1B or the functional fragment or derivative thereof present in the cell.

A substances / test substance / active substance as to be employed for the different aspects of present invention can be any biological or chemical substance or natural product extract, either purified, partially purified, synthesized or manufactured by means of biochemical or molecular biological methods.

A substance able to detectably modulate the CLEC1 B amount or activity is considered a substance active in preventing or treating cardiovascular and/or thrombotic disorders. The detectable amount of CLEC1 B can either refer to a detectable amount CLEC1B nucleic acid (mRNA, cDNA or genomic DNA) and/or protein (prepro/pro/ripe protein). The detectable activity can either refer to transcriptional and/or translational and/or protein activity of CLEC1B DNA/mRNA or protein.

Within the different aspects and embodiments of present invention the term modulation refers to activation or inhibition.

Another example is a method for identifying substances active in preventing or treating cardiovascular and/or thrombotic disorders comprising:
a. Contacting a nucleic acid coding for an CLEC1B protein or a functional fragment or derivative thereof with a test substance in a transcriptionally active system;
b. Determining the amount of mRNA coding for the CLEC1B protein or functional fragment or derivative present in said system in presence of said substance;
c. Determining the amount of mRNA coding for the CLEC1B protein or functional fragment or derivative present in said system in the absence of said substance;
d. Determining whether the substance is capable of modulating the amount of mRNA coding for the CLEC1 B protein or functional fragment or derivative present in said system.

A substance capable of modulating the amount of CLEC1B mRNA present in said system is considered a substance active in preventing or treating cardiovascular and/or thrombotic disorders.

A transcriptionally active system is any biochemical or cellular system, which at least has the ability to perform a transcription reaction of a transcription unit. Such systems are well known in the art and comprise cells (e.g. usual laboratory strains or cell lines as well as primary cultures of eucaryotic or prokaryotic cells) as well as in vitro transcription systems or kits (e.g. on basis of cell extracts) which are also commercially available. In case of present invention this can be a biochemical or cellular system expressing CLEC1 B mRNA or expressing mRNA coding for a functional CLEC1B fragment.

The determination of the mRNA amount present in the system can be performed according to techniques well known in the state of the art (etc. direct labelling of the product by means of radioactive or fluorescent labelling or product detection by use of specific primers or probes etc.).

Another example is a method for identifying substances active in preventing or treating cardiovascular and/or thrombotic disorders comprising:
a. Contacting a nucleic acid coding for an CLEC1B protein or a functional fragment or derivative thereof with a test substance in a translationally active system;
b. Determining the amount of CLEC1B protein or functional fragment or derivative thereof present in said system in presence of said substance;
c. Determining the amount of CLEC1 B protein or functional fragment or derivative thereof present in said system in the absence of said substance;
d. Determining whether the substance is capable of modulating the amount of CLEC 1 B protein or functional fragment or derivative thereof present in said system.

A substance capable of modulating the amount of CLEC1B protein, derivative or fragment present in said system is considered to be a substance active for the prevention or treatment of cardiovascular and/or thrombotic disorders.

A translationally active system is any biochemical or cellular system, which at least has the ability to perform a translation reaction of a transcript. Such systems are well known in the art and comprise cells (e.g. usual laboratory strains or cell lines as well as primary cultures of eucaryotic or prokaryotic cells) as well as in vitro translation systems (which are also commercially available, e.g. as kits). For the in vitro translation of a nucleic acid, the nucleic acid is subcloned in a suitable vector, followed by the expression of the polypeptide in suitable buffers and cell extracts (e.g. reticulocyte lysate). Vectors, necessary reagents and protocols with suitable conditions are known in the art and commercially available.

In the context of present invention, the term "polypeptide° refers to a molecule comprising amino acids bound to each other by peptide bonds and which contain at least 10 amino acids coupled to each other in a linear mode. Shorter molecules of this kind are referred to as peptides. The term "protein° refers to molecules comprising at least one polypeptide chain but can also refer to molecules comprising two or more polypeptide chains associated or bound to each other. Thus, the term "protein" comprises the term "polypeptide".

The detection of the CLEC1B protein present in said system can be performed according to techniques well known in the art (e.g. direct radioactive or fluorescent labelling of the translation product or the employment of specific antibodies, tagging of the protein and detection of the tag, etc.).

Another example concerns a method for identifying substances active in preventing or treating cardiovascular and/or thrombotic disorders comprising:
a. Providing a cell transfected with a nucleic acid vector comprising the promotor of an CLEC1B gene or a functional fragment thereof operationally coupled to a reporter gene or a functional fragment thereof:
b. Providing a cell transfected with a control vector which comprises a reporter gene or a functional fragment thereof not being operationally coupled do a functional CLEC1 B promotor;
c. Determining the reporter gene activity of the cell according to a) and b) in the presence of a test substance;
d. Determining the reporter gene activity of the cell according to a) and b) in absence of the test substance.

Wherein a substance capable of significantly modulating (i.e. increasing or decreasing) reporter gene activity according to a) without significantly modulating reporter gene activity of b) (i.e. capable of specifically increasing CLEC1B promoter activity) is considered to be a substance active in the prevention or treatment of cardiovascular and/or thrombotic disorders.

A significant modulation is any modulation (i.e. increase or decrease) higher than the standard deviation; preferably it is at least two times as high as the standard deviation.

The above example is based on a typical reporter gene assay commonly known in the art. To this end, the promoter of choice is inserted into an expression vector suitable for the type of host cell chosen, upstream of the reporter gene of choice in such a way as to allow for an expression of the reporter gene if the promoter is active. The construct is subsequently introduced into the host cell of choice. Suitable methods for transformation or transfection are well known in the art as well as conditions for cell cultivation and detection of reporter gene expression (see e.g. standard literature listed below). Suitable conditions are well known in the art as well as vectors, reporter genes and necessary reagents, which are also commercially available.

A vector is a circular or linear nucleic acid molecule, e.g. a DNA plasmid, bacteriophage or cosmid, by aid of which nucleic acid fragments (e.g. cut out from other vectors or amplified by PCR and inserted in the cloning vector) can specifically be amplified in suitable cells or organisms. Expression vectors enable the heterologous expression of a gene of interest (e.g. a reporter gene), in the host cell or organism. The type of cell or organism largely depends on the aim and the choice lies within the knowledge of the skilled artisan. Suitable organisms for the amplification of a nucleic acid are e.g. mostly single cell organisms with high proliferation rates, like e.g. bacteria or yeast. Suitable organisms can also be cells isolated and cultivated from multicellular tissues, like e.g. cell lines generated from diverse organisms (e.g. SF9 cells from Spodoptera Frugiperda, etc.). Suitable cloning vectors are known in the art and commercially available at diverse biotech suppliers like, e.g. Roche Diagnostics, New England Biolabs, Promega, Stratagene and many more. Suitable cell lines are e.g. commercially available at the American Type Culture Collection (ATCC).

For the heterologous expression of a protein or polypeptide, the cell can be any prokaryotic or eucaryotic cell suitable for transfection with a nucleic acid vector and of expressing the gene of interest, e.g. a reporter gene. Possible examples thereof are primary cells or cultured cells, preferably eukaryotic cell cultures, which have originally been obtained, for example, from multicellular organisms or tissues (such as, for example, HeLa, CHO, COS, SF9 or 3T3) or which themselves are unicellular organisms, such as, for example, yeast cells (e.g. S. pombe or S. cerevisiae) or prokaryotic cell cultures, or Pichia or E. coli. Cells and samples from tissues may be obtained by known techniques of the prior art (e.g. taking blood samples, tissue puncture or surgical techniques). Suitable for use for the inventive use of CLEC1B are also isolated cells which naturally produce CLEC1 B to determine directly the ability of a cardiovascular pharmaceutical to increase or decrease the amount of CLEC1B produced.

In the context of the present application, the term "transfection" refers to the introduction of a nucleic acid vector into a (pro- or eukaryotic) host cell and thus includes the term "transformation". Said transfection may be stable or transient and can be carried out on the basis of common methods.

The CLEC1B promoter region is the part of the CLEC1B gene, which is capable of controlling transcription of a gene product of interest, if the coding sequence of the gene of interest is cloned into a suitable vector, functionally downstream of the promoter/enhancer, and is transfected into a suitable host cell. The nucleotide sequences upstream of the CLEC1 B gene could be considered as the CLEC1 B promoter region. This region comprises nucleotide sequences downstream of nucleotide number 10043146 of sequence number NC_000012.10.

Functional fragments of the CLEC1B promoter are CLEC1B promoter fragments, which, under the conditions given, can likewise control the transcription of downstream coding sequences.The identification of suitable fragments lies in the skill of the responsible artisan.

A reporter gene can be any gene that allows for an easy quantification of its gene product. A variety of reporter genes for eukaryotic or prokaryotic hosts as well as detection methods and necessary reagents are known in the art and commercially available. These comprise e.g. the genes of beta Lactamase (lacZ), Luciferase, Green or Blue fluorescent protein (GFP or BFP), DsRed, HIS3, URA3, TRP1 or LEU2 or beta Galactosidase. These genes encode proteins, which can be easily detected by means of a visible (colour or luminescent) reaction (e.g. lacZ, Luciferase). These comprise gene-products which can be easily detected by means of a visible (colour or luminescent) reaction or gene-products conferring resistance towards antibiotics like Ampicillin or Kanamycin when expressed. Other reporter gene-products enable the expressing cells to grow under certain conditions like e.g. auxotrophic genes.

A functional fragment of a reporter gene is any fragment of a given reporter gene that allows for an easy quantification of its gene product.

Within the context of the above examples the control vector can be any suitable vector which comprises a reporter gene or functional fragment thereof, but wherein reporter gene expression is not driven by a (functional) CLEC1 B promoter. This can e.g. mean that the reporter gene or functional fragment thereof is not operationally coupled to a functional CLEC1 B promoter (i.e. either totally devoid of a CLEC1 B promoter, comprises a non functional CLEC1 B promoter or promoter fragment or wherein the coupling of promoter and reporter gene is not functional). This can also mean that the reporter gene or functional fragment thereof is operationally coupled to another promoter than the CLEC1 B promoter (e.g. SV40 or another standard promoter). The functional vector and the control vector can also be transfected to the same cell, but in which case the reporter genes need to be different.

Another example concerns a high throughput screen based on a method according to one of the above novel methods for the identification of active substances (such methods are also called "assays").

Analytical methods or analytical systems, so-called assays, which are used to measure the activity or concentration of defined target molecules (so-called targets, mostly proteins or nucleic acids) as parameter for the effectiveness of a potential pharmaceutical compound, are well known in the state of the art. Assays comprise for example biochemical analytical methods or systems using isolated or partly isolated components that are put together to a reaction mixture within a defined space and time, in which the effectiveness of the potential pharmaceutical compounds can be tested (e.g. the above methods). For measuring protease activity, these encompass, e.g. radio isotopic or fluorescent assays for measuring the interaction of a labelled member with a non-labelled member (e.g. the interaction of a labelled substrate and an unlabelled protease, wherein upon cleavage of the substrate part of the labelled substrate is set free; thus the protease activity can be measured by detecting and quantifying the amount of the labelled member set free in a given time). Other examples of assays comprise cell based assays (e.g. the above reporter assays or phenotypical assays, wherein the activity of a substance can be monitored by a change in the phenotype of a cell).

Different types of assays are commonly known in the state of the art and commercially available from commercial suppliers.

According to a further example, a CLEC1 B nucleic acid is used which comprises the position 251 of NM_016509 or a CLEC1 B polypeptide is used which comprises the position 24 of NP_057593.

Since the CLEC1 B gene and protein variant T→C at position 251 of the CLEC1B according to reference sequence NM_016509 (or the corresponding position in the genomic sequence) or Ser→Pro at position 24 of the CLEC1B protein according to reference sequence NP_057593, being most significantly connected with the presence of cardiovascular and/or thrombotic vascular disorders, a preferred embodiment of the present invention relates to the use of a CLEC1B nucleic acid or polypeptide, having one or more of the above polymorphisms, e.g. for performing one or more of the applications according to present invention.

Normally, individual SNPs in the wild type sequence of the gene to be studied are not taken into account in the screening for active compounds by using target genes ("molecular targets"). Since the present application has identified a CLEC1 B variant as being connected with the occurrence of cardiovascular and/or thrombotic disorders, the use of a variant of this kind (in particular against the background of the particular physiological makeup of the cells, which accompanies this) might produce a higher probability of finding active compounds suitable for treating and/or preventing cardiovascular and/or thrombotic vascular disorders. In fact, individuals having a genetic and physiological makeup of this kind are also more likely to suffer from cardiovascular and/or thrombotic disorders. Therefore, the use of CLEC1 B nucleotide sequence with cytidine at position 251 according to reference sequence NM_016509 (or at the corresponding position in the CLEC1 B genomic sequence) should result in finding active compounds to which particular patients having this gene or protein variant respond. The use of the CLEC1 B coding sequence having a T or a C at position 251 according to reference sequence NM_016509 (or at the corresponding position in the CLEC1 B genomic sequence) represent another embodiment of the present invention.

In addition, the SNPs in the CLEC1B gene are suitable for use in active compound screening using targets other than CLEC1 B itself: it is possible to use cells in cellular assays for finding active compounds for the treatment and/or prevention of cardiovascular and/or thrombotic disorders, which compounds have the ability to influence the function and/or activity and/or amount of a target other than CLEC1B, specifically cells whose genome has a defined variant of the CLEC1 B gene with respect to position 251 of the nucleotide sequence according to reference sequence NM_016509 (or at the corresponding position in the CLEC1 B genomic sequence). In this way, it is possible to screen specifically for compounds active in preventing or treating cardiovascular and/or thrombotic disorders, even those that intervene in the function of a gene other than the mutated one, against the genetic background, which is preferably connected with the disease to be treated.

A further example relates to the use of means for the detection of CLEC1 B for diagnosing cardiovascular and/or thrombotic disorders or a predisposition for cardiovascular and/or thrombotic disorders by analyzing a biological sample taken from the body of an individual to be examined.

In this connection, the presence the following variants preferably indicates an increased risk: A CLEC1 B coding region containing a C at position 251 according to reference sequence NM_016509 (or at the corresponding position in the CLEC1B genomic sequence) on at least one allele.

A means for detecting CLEC1B may be any means suitable for detecting a CLEC1B protein or nucleic acid in a biological sample.

The means for detection may be, for example, a means for detecting CLEC1 B mRNA or protein in the sample; e.g. a means on the basis of which the amount of CLEC1B mRNA or protein in a sample can be quantified (for example suitable primers, probes, anti-CLEC1B antibodies, etc.; for example, a nucleic acid probe which is able to hybridize under standard conditions specifically to CLEC1 B mRNA or cDNA. for example for use in a Northern blot or in micro arrays or a Primer set for quantitative Reverse Transcriptase Polymerase Chain Reaction (RT-PCR)). Another example relates to means for determining the type of nucleotide at position 251 according to the reference sequence NM_016509 (or at the corresponding position in the CLEC1B genomic sequence) of the CLEC1B gene, for example a suitable PCR primer set (e.g. genomic or cDNA Primers) to be used e.g. in PCR Sequencing, a probe (to be used, e.g. in Southern blots or chip- or microarray hybridization) or a specific anti-DNA antibody to be used, e.g. in immuno(histo)chemical, -fluorescent or -radiochemical techniques known in the art. Yet another example relates to a means for determining the type of amino acid present at position 24 of the CLEC1B protein according to reference sequence NP_057593. e.g. an antibody specific for a certain protein polymorphism.

According to another embodiment, the means for detection is a means for determining the type of nucleotide at position 251 according to the reference sequence NM_16509 (or at the corresponding position in the CLEC1B genomic sequence) of the CLEC1B gene.

The design and synthesis of suitable primers is known in the prior art; such primers may also be obtained commercially. According to a preferred embodiment, these are the primers according to SEQ ID NOs: 4 and/or 5. Nucleic acids are sequenced by means of conventional routine methods, for example by using customary laboratory robots, which are sold, for example, by companies such as Applied Biosystems, Bio-Rad, etc.

The design and preparation of suitable probes are likewise known in the prior art (see, for example, the standard literature listed).

In a further preferred embodiment of one of the uses or the methods according to the invention, the change in the amount of protein or the determination of a given protein-polymorphism within the CLEC1 B protein is determined with the aid of at least one antibody. Preferred detection methods here are ELISA, Western blot, protein chip and spectrometric methods.

The preparation of suitable antibodies or functional fragments thereof is known in the prior art, for example by immunizing a mammal, for example a rabbit, with CLEC1B protein or a fragment thereof, where appropriate in the presence of a suitable adjuvant (Freund's adjuvant or aluminum hydroxide gel, see, for example, Diamond, B.A. et al. (1981) The New England Journal of Medicine: 1344-1349). The polyclonal antibodies produced in the animal as a result of the immunological reaction may subsequently be isolated and purified by means of known methods, for example by column chromatography. Monoclonal antibodies may be obtained, for example, according to the known method by Winter and Milstein (Winter, G. & Milstein, C. (1991) Nature, 349, 293-299). Suitable methods for preparing and purifying monoclonal antibodies are known in the prior art (see standard literature). Examples of known antibodies for detecting CLEC1B comprise CLEC1B antibodies from Abnova Corporation, Cat.No.: H00051266-A01 or from Novus Biologicals, Catalog Number: H00051266-A01. The use of antibodies for detecting CLEC1 B is known in the art and is also disclosed, e.g. in Fuller et al. or Suzuki-Inoue et al.

In the context of the present group of related inventions, the term antibody or antibody fragment also refers to recombinantly produced antibodies or antigen binding sites thereof, which may also be modified, where appropriate, such as, for example, chimeric antibodies, humanized antibodies, multifunctional antibodies, bi- or oligospecific antibodies or F(ab) or F(ab)₂ fragments.

Conventional immunochemical or immunoradiological methods for detecting an antibody reaction are well known to the skilled worker. Common methods are based, for example, on binding of a specific primary antibody to the antigen to be identified, binding of a secondary antibody, which usually recognizes species-specific epitopes on said primary antibody. Binding of said secondary antibody is utilized here in order to generate a detectable signal (for example a radioactive signal, when radio labeled secondary antibodies are used, or a fluorescent signal, when fluorescence-coupled secondary antibodies are used, or a colorimetrically determinable signal, for example when enzyme-coupled secondary antibodies are used, etc.), see also the literature listed below regarding standard methods.

Another example relates to a diagnostic kit for detecting a predisposition for cardiovascular and/or thrombotic disorders, which kit comprises at least one means for detecting CLEC1 B in biological samples.

In the context of the present invention, the term "kit" (kit of parts) means any combination of components identified herein which have been combined to give a spatially and functionally connected unit for performing a given task (here e.g. for the diagnosis of cardiovascular and/or thrombotic disorders or a predisposition therefore) which may additionally comprise further parts.

A diagnostic kit according to the present invention comprises at least one means for detecting CLEC1B in a biological sample. Suitably, it may furthermore include suitable buffers and/or further reagents for detecting CLEC1 B and/or for preparing or methoding samples and also, where appropriate, instructions for carrying out the particular detection method.

According to a preferred embodiment of the uses or methods according to the invention, the presence of one or more of the variations in the CLEC1 B gene are detected by PCR and, where appropriate, subsequent sequencing or by using a nucleic acid probe. Such a probe can e.g. be a nucleic acid fragment having 50 or more, 100 or more, 150 or more, 200 or more 250 or more, 300 or more, 350 or more, 400 or more contiguous nucleotides according to SEQ ID NO:3, the fragment comprising position 201 of SEQ ID NO:3 (the position, where the SNP according to present invention is located).

Suitable protocols and reagents for PCR or hybridization with suitable probes, which bind, for example, immobilized genomic DNA on suitable supports (e.g. membranes or chips) are well known in the prior art.

A nucleic acid molecule may "hybridize" with another one, if single-stranded forms of both molecules can attach to one another under suitable reaction conditions (temperature and ion concentration of the surrounding medium) in order to form a new double-stranded nucleic acid molecule. In order to hybridize, the nucleic acid molecules attaching to one another must have complementary sequences. However, depending on the chosen stringency conditions, base mismatches are also possible, without stopping an attachment. The term "stringency" describes reaction-conditions, which influence the specificity of hybridization, when two single-stranded nucleic acid molecules attach to one another, and thus also determine how many mismatches or how strong a mismatch between the two molecules are tolerated during attachment. The stringency and thus also the specificity of a reaction here depends inter alia on the temperature and the buffer conditions. Adequate conditions for hybridizing two given nucleic acids, depend on the length, the type of nucleic acid molecules and the degree of complementarity. Said parameters and the determination of suitable conditions for a given analytical method are well known to the skilled person and may also be found in the literature of standard laboratory methods (e.g. "Current Protocols in Molecular Biology", John Wiley & Sons, N.Y. (1989), 6.3.1-6.3.6).

According to a preferred embodiment of the present group of inventions related to one another, the individual is a patient having a cardiovascular and/or thrombotic disease. The cardiovascular disease is preferably a coronary artery disease (>20% stenosis and/or >50% stenosis), myocardial infarction, premature myocardial infarction, acute coronary syndrome or angina pectoris (in particular instable angina).

The isolated sample used for the methods, use or test kit of the present invention is preferably a human sample and the individual to be examined is preferably a human being. Said sample may be in particular: a histological sample, a biopsy sample, a cell (e.g. mucosal cells), a cell extract, cellular tissue, body fluid, preferably blood, saliva, lymph or urine.

The invention additionally relates to uses of isolated CLEC1 B nucleic acids, e.g. nucleic acids having or comprising the sequence or part of the sequence according NM_016509 or according to NT009714 or according to SEQ ID NO:3 or fragments thereof. The nucleic acids or fragments comprising the following SNP:
a. a cytidine at position 251 of the CLEC1B sequence according to NM_016509 (or at the corresponding position in the CLEC1 B genomic sequence, e.g. at position 201 of SEQ ID NO:3) or
b. a thymidine at position 251 of the CLEC1B sequence according to NM_016509 (or at the corresponding position in the CLEC1B genomic sequence, e.g. at position 201 of SEQ ID NO:3).

The invention moreover relates to uses of isolated CLEC1B protein or fragments thereof, the protein or fragments having the following protein polymorphism:
a. a proline at position 24 of the polypeptide chain of the CLEC1B protein according to reference sequence NP_057593 or
b. a serine at position 24 of the polypeptide chain of the CLEC1 B protein according to reference sequence NP_057593.

The invention will be illustrated in more detail below on the basis of examples, which are not to be regarded as limitation, in combination with the figures and tables:

### References:

Chaipan C, Soilleux EJ, Simpson P, Hofmann H, Gramberg T, Marzi A, Geier M, Stewart EA, Eisemann J, SteinkassererA, Suzuki-Inoue K, Fuller GL, Pearce AC, Watson SP, Hoxie JA, Baribaud F, Pohlmann S. DC-SIGN and CLEC-2 mediate human immunodeficiency virus type 1 capture by platelets. J Virol 80: 8951-8960, 2006.
Colonna M, Samaridis J, Angman L. Molecular characterization of two novel C-type lectin-like receptors, one of which is selectively expressed in human dendritic cells. Eur J Immunol 30: 697-704, 2000.
Fuller GL, Williams JA, Tomlinson MG, Eble JA, Hanna SL, Pohlmann S, Suzuki-Inoue K, Ozaki Y, Watson SP, Pearce AC. The C-type lectin receptors CLEC-2 and Dectin-1, but not DC-SIGN, signal via a novel YXXL-dependent signaling cascade. J Biol Chem 282: 12397-12409, 2007.
Kanazawa N. Dendritic cell immunoreceptors: C-type lectin receptors for pattern-recognition and signaling on antigen-presenting cells. J Dermatol Sci 45: 77-86, 2007.
Sobanov Y, Bemreiter A, Derdak S, Mechtcheriakova D, Schweighofer B, Duchler M, Kalthoff F, Hofer E. A novel cluster of lectin-like receptor genes expressed in monocytic, dendritic and endothelial cells maps close to the NK receptor genes in the human NK gene complex. Eur J Immunol 31: 3493-3503, 2001.
Suzuki-Inoue K, Fuller GL, Garcia A, Eble JA, Pohlmann S, Inoue O, Gartner TK, Hughan SC, Pearce AC, Laing GD, Theakston RD, Schweighoffer E, Zitzmann N, Morita T, Tybulewicz VL, Ozaki Y, Watson SP. A novel Syk-dependent mechanism of platelet activation by the C-type lectin receptor CLEC-2. Blood 107: 542-549, 2006.
Watson AA, Brown J, Harlos K, Eble JA, Walter TS, O'Callaghan CA. The crystal structure and mutational binding analysis of the extracellular domain of the platelet-activating receptor CLEC-2. J Biol Chem 282: 3165-3172, 2007.
Standard literature of laboratory methods:
(Unless stated otherwise, the laboratory methods mentioned herein are or can be carried out according to the standard literature listed below.)
Sambrook et al. (1989) Molecular Cloning: A Laboratory Manual. Second edition. Cold Spring Harbor Laboratory Press, Cold Spring Harbor, NY. 545 pp;
Current Protocols in Molecular Biology; regularly updated, e.g. Volume 2000; Wiley & Sons, Inc; Editors: Fred M. Ausubel, Roger Brent, Robert Eg. Kingston, David D. Moore, J.G. Seidman, John A. Smith, Kevin Struhl.
Current Protocols in Human Genetics; regularly updated; Wiley & Sons, Inc; Editors: Nicholas C. Dracopoli, Honathan L. Haines, Bruce R. Korf, Cynthia C. Morton, Christine E. Seidman, J.G. Seigman, Douglas R. Smith.
Current Protocols in Protein Science; regularly updated; Wiley & Sons, Inc; Editors: John E. Coligan, Ben M. Dunn, Hidde L. Plögh, David W. Speicher, Paul T. Wingfield.
Molecular Biology of the Cell; third edition; Alberts, B., Bray, D., Lewis, J., Raff, M., Roberts, K., Watson, J.D.; Garland Publishing, Inc. New York & London, 1994;
Short Protocols in Molecular Biology, 5th edition, by Frederick M. Ausubel (Editor), Roger Brent (Editor), Robert E. Kingston (Editor), David D. Moore (Editor), J.G. Seidman (Editor), John A. Smith (Editor), Kevin Struhl (Editor), October 2002, John Wiley & Sons, Inc., New York
Transgenic Animal Technology A Laboratory Handbook. C.A. Pinkert, editor; Academic Press Inc., San Diego, California, 1994 (ISBN: 0125571658)
Gene targeting: A Practical Approach, 2nd Ed., Joyner AL, ed. 2000. IRL Press at Oxford University Press, New York;
Manipulating the Mouse Embryo: A Laboratory Manual. Nagy, A, Gertsenstein, M., Vintersten, K., Behringer, R., 2003, Cold Spring Harbor Press, New York;

### Example:

1. SNP Detection by sequencing and analyses of sequencing results
1 a) Amplification of DNA regions in the CLEC1B gene Oligonucleotides (primers) for DNA amplification:
For detection of the nucleotide present at position 251 in the CLEC1B gene according to reference sequence NM_016509 (or at the corresponding position in the CLEC1B genomic sequence according to NT009714), one or both of the following primers could be used:
Primer 1 (forward primer): 5'- TGCCTGCTCCTTGATGTCTTTATT -3' (SEQ ID NO: 4)
Primer 2 (reverse primer): 5'- TCAGCAGAATCAAAGCCATCACA-3' (SEQ ID NO: 5)

PCR protocol for amplification:
Reagents used are from Applied Biosystems (Foster City, USA):
20 ng of genomic DNA; 1 unit of TaqGold DNA polymerase; 1 × Taq polymerase buffer; 500 µM dNTPs; 2.5 mM MgCl₂; 200 nM of each amplification primer pair (sequences under 1.A); H₂O to 5 µl.

PCR amplification program for genotyping:

| | |
|---|---|
| 95°C for 10 min | × 1 cycle; |
| | |
| 95°C for 30 s | |
| 70°C for 30 s | × 2 cycles; |
| | |
| 95°C for 30 s | |
| 65°C for 30 s | × 2 cycles; |
| | |
| 95°C for 30 s | |
| 60°C for 30 s | × 2 cycles; |
| | |
| 95°C for 30 s | |
| 56°C for 30 s | |
| 72°C for 30 s | × 40 cycles; |
| | |
| 72°C for 10 min | |
| 4°C for 30 s | × 1 cycle. |

1b) Identification of SNPs Protocol for minisequencing and detection of SNPs

All reagents are from Applied Biosystems (Foster City, USA). 2 µl of purified PCR product, 1.5 µl of BigDye terminator kit, 200 nM sequencing primer (for sequences, see under 1.A), H₂O to 10 µl.

Amplification program for sequencing:

| | |
|---|---|
| 96°C for 2 min | × 1 cycle; |
| | |
| 96°C for 10 s | |
| 55°C for 10 s | |
| 65°C for 4 min | × 30 cycles; |
| | |
| 72°C for 7 min | |
| 4°C for 30 s | × 1 cycle. |

### Example 2: Statistical analysis of the identified SNPs

The analyzed CLEC1 B polymorphism in the reference nucleotide sequences NM_016509. NT009714 and in the reference protein sequence NP_057593 was analyzed for association with clinical parameters in about 1400 individuals. The characteristics of the analyzed cohort are listed in table 1. The frequency and distribution of the different identified polymorphisms is shown in table2. Associations of the CLEC2 polymorphism Ser→Pro at position 24 of the reference sequence NP_057593 with clinical endpoints in the patient group analyzed can be found in tables 3, 4 and 5. All statistical analyses were carried out with SAS version 8.2 (SAS Institute GmbH, Heidelberg, Germany).

The p value is a parameter relating to the statistical significance of the associations observed. RR (risk ratio) is a parameter relating to the increased risk of the occurrence of the clinical end point indicated in patients having a certain CLEC1B polymorphism. RR was calculated with adjustment of the patient groups with respect to age, gender, smoker status, blood pressure and diabetes status.

As shown in Table 3, individuals with CLEC1B-C251C, who are homozygous for Pro25Pro have greater risk of experiencing coronary artery disease (CAD), with a stenosis >20%, than individuals with CLEC1B-T251T (Ser24Ser). A dependency on the presence of C (cytidin) in position 251 of the CLEC1B nucleotide sequence on the frequency of CAD can be observed, since the frequency of having CAD increases with the number of C alleles in the individuals. That is, a CAD frequency of 76.89% for individuals without C allele, a CAD frequency of 84.24% for individuals with one C allele and a CAD frequency of 88.89% for individuals with two C alleles in position 251 of the CLEC1B nucleotide sequence. Furthermore, individuals with CLEC1B-C251C, who are homozygous for Pro25Pro have greater risk of experiencing coronary artery disease (CAD), with a stenosis >50%, than individuals with CLEC1B-T251T (Ser24Ser). A dependency on the presence of C (cytidin) in position 251 of the CLEC1 B nucleotide sequence on the frequency of CAD can be observed, since the frequency of having CAD increases with the number of C alleles in the individuals. That is, a CAD frequency of 66.35% for individuals without C allele, a CAD frequency of 74.50% for individuals with one C allele and a CAD frequency of 83.33% for individuals with two C alleles in position 251 of the CLEC1B nucleotide sequence.

As outlined in Tables 4 and 5, the statistically significant results for an association of the CLEC1B polymorphism in position 24 of the protein (or position 251 of the nucleotide sequence) with coronary artery disease is independent of confounding factors such as gender, diabetes and smoker status or hypertension. Individuals with CLEC1B-C251C (Pro24Pro) have 1.610 (p-value=0.0026) and 1.499 (p-value=0.0021) times elevated risk of getting coronary artery disease with >20% stenosis and >50% stenosis, respectively.

As one outcome of said study it can be generally stated that:
Cytidine at position 251 in the CLEC1B coding region according to reference sequence NM_016509 on one or both alleles increases significantly the risk of experiencing or developing a cardiovascular and/or thrombotic disorder,
especially a coronary artery disease, potentially necessitating therapeutic intervention, especially in individuals not having a thymidine at position 251 in the CLEC1B nucleotide sequence according to reference sequence NM_016509.

The associations between the clinical endpoints and genetic variation in the CLEC1B gene and/or protein are a clear hint to the impact of the genetic variant Ser→Pro at position 24 in the CLEC1B protein on the onset of cardiovascular and/or thrombotic disorders such as coronary artery disease with stenosis of >20% and >50% of the vessel lumen. The statistically significant correlation between the variant of CLEC1B and said clinical endpoints demonstrated here for the first time thus provides a meaningful basis for present invention.

### Legend to figures and tables:

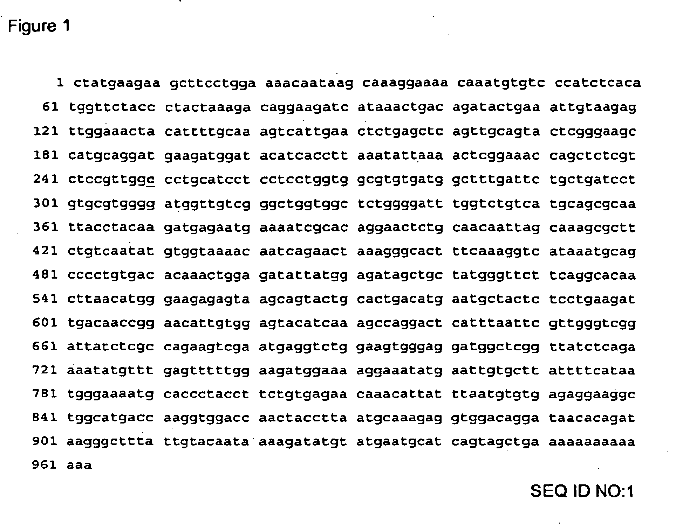

Figure 1: CLEC1B coding sequence with the NCBI reference number NM_016509 (SEQ ID NO:1). The polymorphism at position 251 is underlined and marked in bold.

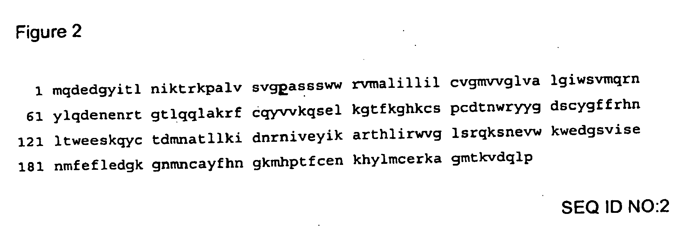

Figure 2: CLEC1 B protein sequence with the NCBI reference number NP_057593 (SEQ ID NO:2). The polymorphism at position 24 is marked in bold.

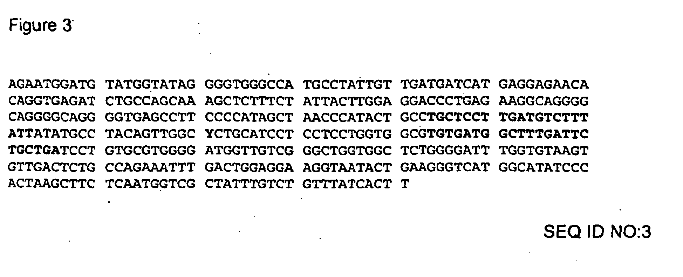

Figure 3: Nucleic acid fragment of CLEC1 B genomic DNA as disclosed under accession number rs_2273986 in the NCBI database (SEQ ID NO:3) containing the SNP according to present invention. The position of the PCR primers and the polymorphism are marked bold. "Y" stands for C or T.

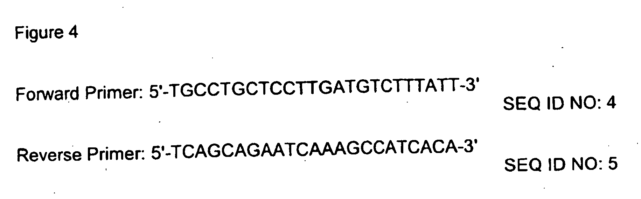

Figure 4: PCR Primers for analyzing the type of nucleotide present at position 251 with respect to reference sequence NM_016509 or the respective position of the genomic sequence (SEQ ID NOs: 4 and 5).

### Tables:

### Abbreviations:

Ser = Serine
Pro = Proline
CAD = coronary artery disease
CAD20 = 20% or more visual stenosis
CAD50 = 50% or more visual stenosis

**Table 1:** Basic characteristics of patient cohort
**Table 2:** Distribution of CLEC1B polymorphism Ser24Pro in LURIC cohort
**Table 3:** Association of CLEC1 B polymorphism Ser24Pro with coronary artery disease in LURIC cohort
**Table 4:** Analysis of the influence of (Ser→Pro) polymorphism at position 24 of the CLEC1B protein on coronary artery disease with 20% or more stenosis in LURIC cohort including confounding factors (logistic regression). CAD20 = 20% or more visual stenosis, CAD50 = 50% or more visual stenosis.

### Tables:

Abbreviations:
Ser = Serine
Pro = Proline
CAD = coronary artery disease
CAD20 = 20% or more visual stenosis
CAD50 = 50% or more visual stenosis

**Table 1: Basic characteristics of patient cohort**

| | | |
|---|---|---|
| Age [years] | | 61.79 ± 10.57 |
| BMI [kg] | | 27.97 ± 4.41 |
| Gender | male | 1016 (70.75%) |
| | female | 420 (29.25%) |
| | | |
| Diabetes (yes) (*) | | 595 (31.27%) |
| Hypertension (yes) (**) | | 1123 (58.34%) |
| Smoker (yes) | | 1277 (66.34%) |
| CAD (yes) | | 1367 (78.65%) |
| Myocardial Infarction (yes) | | 802 (42.14%) |

| | | |
|---|---|---|
| (*) all diabetics according to ADA criteria (**) arterial hypertension | | |

**Table 2: Distribution of CLEC1B polymorphism Ser24Pro in LURIC cohort**

| | n | % |
|---|---|---|
| Ser24Ser | 1061 | 73.89 |
| Ser24Pro | 357 | 24.86 |
| Pro24Pro | 18 | 1.25 |

**Table 3: Association of CLEC1B polymorphism Ser24Pro with coronary artery disease in LURIC cohort**

| | | Ser24Ser | Ser24Pro | Pro24Pro | |
|---|---|---|---|---|---|
| | | n (%) | n (%) | n (%) | p-value |
| CAD20 | yes | 802 (76.89) | 294 (84.24) | 16 (88.89) | 0.0084 |
| | no | 259 (23.11) | 63(15.76) | 2 (11.11) | |
| CAD50 | yes | 692 (66.35) | 260 (74.50) | 15 (83.33) | 0.0071 |
| | no | 369 (33.65) | 97 (25.50) | 3 (16.67) | |

**Table 4: Analysis of the influence of (Ser→Pro) polymorphism at position 24 of the CLEC1B protein on coronary artery disease with 20% or more stenosis in LURIC cohort including confounding factors (logistic regression). CAD20 = 20% or more visual stenosis, CAD50 = 50% or more visual stenosis.**

| | | |
|---|---|---|
| CLEC1 B Ser→Pro polymorphism | 0.0026 | 1.610 (1.181-2.198) |
| Gender | <0.0001 | 2.584 (1.908-3.497) |
| Type II diabetes (*) | <0.0001 | 2.198 (1.577-3.058) |
| Smoker status | 0.0005 | 1.695 (1.258-2.283) |
| Hypertension (**) | <0.0001 | 2.273 (1.721-2.994) |

**Table 5: Analysis of the influence of (Ser→Pro) polymorphism at position 24 of the CLEC1B protein on coronary artery disease with 50% or more stenosis in LURIC cohort including confounding factors (logistic regression). CAD20 = 20% or more visual stenosis, CAD50 = 50% or more visual stenosis.**

| | | |
|---|---|---|
| CLEC1B Ser-Pro polymorphism | 0.0021 | 1.499 (1.152-1.942) |
| Gender | <0.0001 | 2.639 (2.012-3.448) |
| Type II diabetes (*) | <0.0001 | 1.812 (1.379-2.381) |
| Smoker status | <0.0001 | 1.764-(1.355-2.294) |
| Hypertension (**) | <0.0001 | 1.603 (1.256-2.041) |

## Claims

1. A method for the identification of cardiovascular and/or thrombotic disorders or of an increased risk for developing cardiovascular and/or thrombotic disorders in a biological sample taken from an individual to be examined, wherein the SNP at position 251 of the CLEC1B gene according to SEQ ID NO 1 is analyzed.

2. A method for the identification of cardiovascular and/or thrombotic disorders or of an increased risk for developing cardiovascular and/or thrombotic disorders in a biological sample taken from an individual to be examined, by analyzing a CLEC1B nucleic acid for the presence of the single nucleotide polymorphism thymidine (T) → cytidine (C) at position 251 of the CLEC1B nucleic acid sequence according to reference sequence SEQ ID No 1 and/or by analyzing a CLEC1 B protein for the presence of the protein polymorphisms serine (Ser) → proline (Pro) at position 24 of the CLEC1B protein according to reference sequence SEQ ID No 2.

3. A method for identifying cardiovascular and/or thrombotic disorders or an increased risk for developing cardiovascular and/or thrombotic disorders in an individual, which comprises examining a sample taken from an individual
a) for the type of nucleotide, which is present at the position 251 on one or both alleles of the CLEC1B gene according to SEQ ID No 1, the presence of a cytidine nucleotide at said position being indicative for an increased risk of said individual to suffer from or develop cardiovascular and/or thrombotic disorders, the presence of a nucleotide other than cytidine, preferably a thymidine indicating a lowered risk of said individual to suffer from or develop cardiovascular and/or thrombotic disorders; or
b) for the type of amino acid present at position 24 of the polypeptide chain of CLEC1 B protein according to SEQ ID No 2, the presence of a proline at said position being indicative of an increased risk of said individual to suffer from or develop cardiovascular and/or thrombotic disorders, the presence of an amino acid other than proline, preferably a serine at position 24 of the CLEC1B protein according to SEQ ID NO 2 indicating a lowered risk of said individual to suffer from or develop cardiovascular and/or thrombotic disorders.

4. A method of determining the risk of suffering from cardiovascular and/or thrombotic disorders comprising analyzing an isolated sample of an individual for the presence of one or more of the SNPs or protein polymorphisms as identified in claims 1-3 and calculating the estimated risk on basis of the age and the type of nucleotide or amino acid present at position 251 of the CLEC1B gene according to SEQ ID NO 1 or at position 24 of the CLEC1 B protein according to SEQ ID NO 2.

5. A method for adapting the dosage of a pharmaceutical for the prevention and/or treatment of cardiovascular and/or thrombotic disorders, wherein one or more of the SNPs at position 251 of the CLEC1 B gene according to SEQ ID NO 1 is analyzed.

6. A method of for adapting the dosage of a pharmaceutical for the prevention and/or treatment of cardiovascular and/or thrombotic disorders, by analyzing a CLEC1 B nucleic acid for the presence of the single nucleotide polymorphism thymidine (T) → cytidine (C) at position 251 of the CLEC1B nucleic acid sequence according to reference sequence SEQ ID No 1 and/or by analyzing a CLEC1B protein for the presence of the protein polymorphisms serine (Ser) → proline (Pro) at position 24 of the CLEC1 B protein according to reference sequence SEQ ID No 2.

7. A method for adapting the dosage of a pharmaceutical for the prevention and/or treatment of cardiovascular and/or thrombotic disorders in an individual, which method comprises examining a taken sample of the individual
a) for the type of the nucleotide, which is present at position 251 on either or both alleles of the CLEC1 B gene according to SEQ ID NO 1, said dosage being adapted dependent of the presence of a cytidine or thymidine nucleotide at one or more of said positions, the presence of one or more of said variants indicating the dosage being adapted; or
b) for the type of amino acid present at position 24 in the CLEC1B protein according to SEQ ID NO 2, said dosage being adapted dependent of the presence of a proline or serine at said position: the dosage of the pharmaceutical being adapted in the presence of one or more of said genomic variants; or
c) as to whether the amount of CLEC1B mRNA and/or protein present in said sample is different from that of one or more taken reference samples said dosage being adapted depending on whether the amount of protein and/or mRNA in the taken sample of the individual is different from that of the reference sample or reference samples.

8. A method for identifying individuals responding to a pharmaceutical for the treatment and/or prevention of cardiovascular and/or thrombotic disorders, wherein the SNP at position 251 of the CLEC1B gene according to SEQ ID NO 1 is analyzed.

9. A method for identifying individuals responding to a pharmaceutical for the treatment and/or prevention of cardiovascular and/or thrombotic disorders, by analyzing a CLEC1 B nucleic acid for the presence of the single nucleotide polymorphism thymidine (T) → cytidine (C) at position 251 of the CLEC1B nucleic acid sequence according to reference sequence SEQ ID No 1 and/or by analyzing a CLEC1 B protein for the presence of the protein polymorphisms serine (Ser) → proline (Pro) at position 24 of the CLEC1 B protein according to reference sequence SEQ ID No 2.

10. The method according to any of the preceding claims, wherein the cardiovascular and/or thrombotic disorders is angina pectoris, instable angina pectoris, heart infarction, early heart infarction, peripheral vascular disorder, coronary heart disease, high blood pressure, stroke, PRIND, TIA or a disorder necessitating the undertaking of a coronary angioplasty.

11. The method as claimed in any of the preceding claims, comprising analyzing a taken sample of an individual, wherein the individual whose taken sample is examined has a glucose metabolism disorder, preferably suffering from diabetes and particularly preferably from type I diabetes.

12. The method as claimed in any of the preceding claims, wherein the individual whose taken sample is examined suffers from cardiovascular and/or thrombotic disorders.

13. The method as claimed in any of the preceding claims, wherein the sample is a mammalian and preferably a human sample.

14. The method as claimed in any of the preceding claims, wherein the sample is a histological sample, a biopsy sample, a cell extract, one or more cells or taken body fluid.

15. The method according to any of the preceding claims 1 to 9, wherein CLEC1B is used as an isolated molecule.

16. The method according to one of the claims 16 or 17, wherein the SNPs are: a cytidine at position 251 of the CLEC1B sequence on one or both alleles of the CLEC1B gene (according to SEQ ID NO 2);

17. The method according to claim 1-4 or 6, wherein the identification of the following protein polymorpisms indicates the disease or an increased risk: a proline at position 24 of the CLEC1B protein according to SEQ ID NO 2;

18. The method as claimed in any of claims 1-3 or 6-10, wherein the type of nucleotide at position 251 in the CLEC1 B gene according to SEQ ID NO 1 is determined by means of one or more suitable primers or probes.

19. The method as claimed in claim 20 wherein the type of nucleotide is identified by means of PCR, Southern Blot, Array- or Chip-hybridization.

20. The method as claimed in claim 7, wherein the change in the amount of mRNA is analyzed, preferably using one or more suitable primers for the amplification of CLEC1B cDNA or one or more probes suitable for hybridization with CLEC1B cDNA or mRNA at standard conditions.

21. The method according to one of the claims 20 to 23, **characterized by** a primer set containing one or both primers of the sequence as defined in SEQ ID NOs: 4 and 5 or a probe as defined in the sequence SEQ ID NO:3.

22. A nucleic acid primer with a nucleic acid sequence according to SEQ ID NO: 4 or 5.

23. A nucleic acid probe with the nucleic acid sequence according to SEQ ID NO: 3.

## Patentansprüche

1. Verfahren zur Identifizierung von Herz-Kreislauf- und/oder thrombotischen Erkrankungen oder eines erhöhten Risikos der Entwicklung von Herz-Kreislauf- und/oder thrombotischen Erkrankungen in einer einem zu untersuchenden Individuum entnommenen biologischen Probe, wobei der SNP an Position 251 des CLEC1B-Gens gemäß SEQ ID NO 1 analysiert wird.

2. Verfahren zur Identifizierung von Herz-Kreislauf- und/oder thrombotischen Erkrankungen oder eines erhöhten Risikos der Entwicklung von Herz-Kreislauf- und/oder thrombotischen Erkrankungen in einer einem zu untersuchenden Individuum entnommenen biologischen Probe durch Analysieren einer CLEC1B-Nukleinsäure auf das Vorliegen des Einzelnukleotid-Polymorphismus Thymidin (T) → Cytidin (C) an Position 251 der CLEC1B-Nukleinsäuresequenz gemäß Referenzsequenz SEQ ID NO 1 und/oder Analysieren eines CLEC1B-Proteins auf das Vorliegen des Protein-Polymorphismus Serin (Ser) → Prolin (Pro) an Position 24 des CLEC1B-Proteins gemäß Referenzsequenz SEQ ID NO 2.

3. Verfahren zum Identifizieren von Herz-Kreislauf- und/oder thrombotischen Erkrankungen oder eines erhöhten Risikos der Entwicklung von Herz-Kreislauf- und/oder thrombotischen Erkrankungen bei einem Individuum, wobei man eine einem Individuum entnommene Probe
a) auf die Nukleotidart, die an Position 251 auf einem Allel oder beiden Allelen des CLEC1B-Gens gemäß SEQ ID NO 1 vorliegt, untersucht, wobei das Vorliegen eines Cytidinnukleotids an der Position ein erhöhtes Risiko für das Individuum, an Herz-Kreislauf- und/oder thrombotischen Erkrankungen zu leiden oder diese zu entwickeln, anzeigt, wobei das Vorliegen eines anderen Nukleotids als Cytidin, vorzugsweise eines Thymidins, ein verringertes Risiko für das Individuum, an Herz-Kreislauf- und/oder thrombotischen Erkrankungen zu leiden oder diese zu entwickeln, anzeigt; oder
b) auf die an Position 24 der Polypeptidkette des CLEC1B-Proteins gemäß SEQ ID NO 2 vorliegende Aminosäureart untersucht, wobei das Vorliegen eines Prolins an der Position ein erhöhtes Risiko für das Individuum, an Herz-Kreislauf- und/oder thrombotischen Erkrankungen zu leiden oder diese zu entwickeln, anzeigt, wobei das Vorliegen einer anderen Aminosäure als Prolin, vorzugsweise eines Serins, an Position 24 des CLEC1B-Proteins gemäß SEQ ID NO 2 ein verringertes Risiko für das Individuum, an Herz-Kreislauf- und/oder thrombotischen Erkrankungen zu leiden oder diese zu entwickeln, anzeigt.

4. Verfahren zur Bestimmung des Risikos, an Herz-Kreislauf- und/oder thrombotischen Erkrankungen zu leiden, wobei man eine isolierte Probe eines Individuums auf das Vorliegen eines oder mehrerer der SNPs oder Protein-Polymorphismen mit der in Anspruch 1-3 angegebenen Bedeutung analysiert und das geschätzte Risiko auf der Grundlage des Alters und der an Position 251 des CLEC1B-Gens gemäß SEQ ID NO 1 oder an Position 24 des CLEC1B-Proteins gemäß SEQ ID NO 2 vorliegenden Nukleotid- oder Aminosäureart berechnet.

5. Verfahren zur Anpassung der Dosierung eines Arzneimittels zur Vorbeugung und/oder Behandlung von Herz-Kreislauf- und/oder thrombotischen Erkrankungen, wobei einer oder mehrere der SNPs an Position 251 des CLEC1B-Gens gemäß SEQ ID NO 1 analysiert wird bzw. werden.

6. Verfahren zur Anpassung der Dosierung eines Arzneimittels zur Vorbeugung und/oder Behandlung von Herz-Kreislauf- und/oder thrombotischen Erkrankungen durch Analysieren einer CLEC1B-Nukleinsäure auf das Vorliegen des Einzelnukleotid-Polymorphismus Thymidin (T) → Cytidin (C) an Position 251 der CLEC1B-Nukleinsäuresequenz gemäß Referenzsequenz SEQ ID NO 1 und/oder Analysieren eines CLEC1B-Proteins auf das Vorliegen des Protein-Polymorphismus Serin (Ser) → Prolin (Pro) an Position 24 des CLEC1B-Proteins gemäß Referenzsequenz SEQ ID NO 2.

7. Verfahren zur Anpassung der Dosierung eines Arzneimittels zur Vorbeugung und/oder Behandlung von Herz-Kreislauf- und/oder thrombotischen Erkrankungen bei einem Individuum, wobei man bei dem Verfahren eine entnommene Probe des Individuums
a) auf die Nukleotidart, die an Position 251 auf einem Allel oder beiden Allelen des CLEC1B-Gens gemäß SEQ ID NO 1 vorliegt, untersucht, wobei die Dosierung je nach Vorliegen eines Cytidin- oder Thymidinnukleotids an einer oder mehreren der Positionen angepasst wird, wobei das Vorliegen einer oder mehrerer der Varianten das Anpassen der Dosierung anzeigt; oder
b) auf die an Position 24 in dem CLEC1B-Protein gemäß SEQ ID NO 2 vorliegende Aminosäureart untersucht, wobei die Dosierung je nach Vorliegen eines Prolins oder Serins an der Position angepasst wird: wobei die Dosierung des Arzneimittels in Gegenwart einer oder mehrerer der genomischen Varianten angepasst wird; oder
c) darauf, ob sich die Menge an in der Probe vorliegender CLEC1B-mRNA und/oder vorliegendem CLEC1B-Protein von der einer oder mehrerer entnommener Referenzproben unterscheidet, untersucht, wobei die Dosis je nachdem, ob sich die Menge an Protein und/oder mRNA in der entnommenen Probe des Individuums von der der Referenzprobe oder Referenzproben unterscheidet, angepasst wird.

8. Verfahren zum Identifizieren von Individuen, die auf ein Arzneimittel zur Behandlung und/oder Vorbeugung von Herz-Kreislauf- und/oder thrombotischen Erkrankungen ansprechen, wobei der SNP an Position 251 des CLEC1B-Gens gemäß SEQ ID NO 1 analysiert wird.

9. Verfahren zum Identifizieren von Individuen, die auf ein Arzneimittel zur Behandlung und/oder Vorbeugung von Herz-Kreislauf- und/oder thrombotischen Erkrankungen ansprechen, durch Analysieren einer CLEC1B-Nukleinsäure auf das Vorliegen des Einzelnukleotid-Polymorphismus Thymidin (T) → Cytidin (C) an Position 251 der CLEC1B-Nukleinsäuresequenz gemäß Referenzsequenz SEQ ID NO 1 und/oder Analysieren eines CLEC1B-Proteins auf das Vorliegen des Protein-Polymorphismus Serin (Ser) → Prolin (Pro) an Position 24 des CLEC1B-Proteins gemäß Referenzsequenz SEQ ID NO 2.

10. Verfahren gemäß einem der vorhergehenden Ansprüche, wobei es sich bei den Herz-Kreislauf- und/oder thrombotischen Erkrankungen um Angina pectoris, instabile Angina pectoris, Herzinfarkt, frühzeitigen Herzinfarkt, periphere Gefäßkrankheit, koronare Herzkrankheit, Bluthochdruck, Schlaganfall, PRIND, TIA oder eine Erkrankung, die die Durchführung einer Koronarangioplastie notwendig macht, handelt.

11. Verfahren nach einem der vorhergehenden Ansprüche, bei dem man eine entnommene Probe eines Individuums analysiert, wobei das Individuum, dessen entnommene Probe untersucht wird, eine Glucosestoffwechselstörung hat und dabei vorzugsweise an Diabetes und besonders bevorzugt an Typ-I-Diabetes leidet.

12. Verfahren nach einem der vorhergehenden Ansprüche, wobei das Individuum, dessen entnommene Probe untersucht wird, an Herz-Kreislauf- und/oder thrombotischen Erkrankungen leidet.

13. Verfahren nach einem der vorhergehenden Ansprüche, wobei es sich bei der Probe um eine Säugerprobe und vorzugsweise eine menschliche Probe handelt.

14. Verfahren nach einem der vorhergehenden Ansprüche, wobei es sich bei der Probe um eine histologische Probe, eine Biopsieprobe, einen Zellextrakt, eine oder mehrere Zellen oder entnommene Körperflüssigkeit handelt.

15. Verfahren gemäß einem der vorhergehenden Ansprüche 1 bis 9, wobei CLEC1B als isoliertes Molekül verwendet wird.

16. Verfahren gemäß einem der Ansprüche 16 oder 17, wobei es sich bei den SNPs um ein Cytidin an Position 251 der CLEC1B-Sequenz auf einem Allel oder beiden Allelen des CLEC1B-Gens (gemäß SEQ ID NO 2) handelt.

17. Verfahren gemäß Anspruch 1-4 oder 6, wobei die Identifizierung der folgenden Protein-Polymorphismen die Krankheit oder ein erhöhtes Risiko anzeigt: eines Prolins an Position 24 des CLEC1B-Proteins (gemäß SEQ ID NO 2).

18. Verfahren nach einem der Ansprüche 1-3 oder 6-10, wobei die Nukleotidart an Position 251 im CLEC1B-Gen gemäß SEQ ID NO 1 mittels eines oder mehrerer geeigneter Primer oder einer oder mehrerer geeigneter Sonden bestimmt wird.

19. Verfahren nach Anspruch 20, wobei die Nukleotidart mittels PCR, Southern Blot, Array- oder Chip-Hybridisierung identifiziert wird.

20. Verfahren nach Anspruch 7, wobei die Änderung der Menge an mRNA analysiert wird, wobei man vorzugsweise einen oder mehrere geeignete Primer zur Amplifikation von CLEC1B-cDNA oder eine oder mehrere zur Hybridisierung mit CLEC1B-cDNA oder -mRNA bei Standardbedingungen geeignete Sonden einsetzt.

21. Verfahren gemäß einem der Ansprüche 20 bis 23, **gekennzeichnet durch** einen einen oder beide Primer der wie in SEQ ID NO: 4 und 5 angegebenen Sequenz enthaltenden Primersatz oder eine wie in der Sequenz SEQ ID NO: 3 angegebene Sonde.

22. Nukleinsäure-Primer mit einer Nukleinsäuresequenz gemäß SEQ ID NO: 4 oder 5.

23. Nukleinsäuresonde mit der Nukleinsäuresequenz gemäß SEQ ID NO: 3.

## Revendications

1. Procédé pour l'identification de troubles cardiovasculaires et/ou thrombotiques ou d'un risque accru de développement de troubles cardiovasculaires et/ou thrombotiques dans un échantillon biologique prélevé d'un individu à examiner, dans lequel c'est le SNP sur la position 251 du gène CLEC1B selon SEQ ID N° 1 qui est analysé.

2. Procédé pour l'identification de troubles cardiovasculaires et/ou thrombotiques ou d'un risque accru de développement de troubles cardiovasculaires et/ou thrombotiques dans un échantillon biologique prélevé d'un individu à examiner, par analyse d'un acide nucléique du CLEC1B portant sur la présence d'un polymorphisme mononucléotidique thymidine (T) → cytidine (C) sur la position 251 de la séquence d'acide nucléique de CLEC1B selon la séquence de référence SEQ ID N° 1 et/ou par analyse d'une protéine CLEC1B pour ce qui est de la présence des polymorphismes protéiques sérine (Ser) → proline (Pro) sur la position 24 de la protéine CLEC1B selon la séquence de référence SEQ ID N° 2.

3. Procédé pour identifier des troubles cardiovasculaires et/ou thrombotiques ou un risque accru de développement de troubles cardiovasculaires et/ou thrombotiques chez un individu, qui comprend l'examen d'un échantillon prélevé d'un individu
a) pour ce qui est du type de nucléotide qui est présent sur la position 251 sur un allèle ou sur les deux allèles du gène CLEC1B selon SEQ ID N° 1, la présence d'un nucléotide cytidine sur cette position étant indicative d'un risque accru, pour cet individu, de souffrir de troubles cardiovasculaires et/ou thrombotiques, ou de les développer, la présence d'un nucléotide autre que la cytidine, de préférence une thymidine étant indicative d'un risque réduit, pour ledit individu, de souffrir de troubles cardiovasculaires et/ou thrombotiques, ou de les développer ; ou
b) pour ce qui est du type d'acide aminé présent sur la position 24 de la chaîne polypeptidique de la protéine CLEC1B selon SEQ ID N° 2, la présence d'une proline sur ladite position étant indicative d'un risque accru, pour ledit individu, de souffrir de troubles cardiovasculaires et/ou thrombotiques ou de les développer, la présence d'un acide aminé autre que la proline, de préférence une sérine, sur la position 24 de la protéine CLEC1B selon SEQ ID N° 2 étant indicative d'un risque réduit, pour ledit individu, de souffrir de troubles cardiovasculaires et/ou thrombotiques ou de les développer.

4. Procédé de détermination du risque de souffrir de troubles cardiovasculaires et/ou thrombotiques, comprenant l'analyse d'un échantillon isolé d'un individu pour ce qui est de la présence d'un ou plusieurs des SNP ou des polymorphismes protéiques tels qu'identifiés dans les revendications 1 à 3, et le calcul du risque estimé, sur la base de l'âge et du type de nucléotide ou d'acide aminé présent sur la position 251 du gène CLEC1B selon SEQ ID N° 1 ou sur la position 24 de la protéine CLEC1B selon SEQ ID N° 2.

5. Procédé pour adapter la posologie d'un produit pharmaceutique à la prévention et/ou au traitement de troubles cardiovasculaires et/ou thrombotiques, dans lequel on analyse un ou plusieurs des SNP sur la position 251 du gène CLEC1B selon SEQ ID N° 1.

6. Procédé pour adapter la posologie d'un produit pharmaceutique à la prévention et/ou au traitement de troubles cardiovasculaires et/ou thrombotiques, par analyse d'un acide nucléique de CLEC1B pour ce qui est de la présence d'un polymorphisme mononucléotidique thymidine (T) → cytidine (C) sur la position 251 de la séquence de l'acide nucléique de CLEC1B selon la séquence de référence SEQ ID N° 1 et/ou par analyse d'une protéine CLEC1B pour ce qui est de la présence des polymorphismes protéiques sérine (Ser) → proline (Pro) sur la position 24 de la protéine CLEC1B selon la séquence de référence SEQ ID N° 2.

7. Procédé pour adapter la posologie d'un produit pharmaceutique à la prévention et/ou au traitement de troubles cardiovasculaires et/ou thrombotiques chez un individu, ce procédé comprenant l'examen d'un échantillon prélevé de l'individu
a) pour ce qui est du type du nucléotide qui est présent sur la position 251 sur l'un ou l'autre des allèles, ou les deux, du gène CLEC1B selon SEQ ID N° 1, ladite posologie étant adaptée en fonction de la présence d'un nucléotide cytidine ou thymidine sur une ou plusieurs desdites positions, la présence d'un ou plusieurs desdits variants indiquant la posologie adaptée ; ou
b) pour ce qui est du type d'acide aminé présent sur la position 24 de la protéine CLEC1B selon SEQ ID N° 2, ladite posologie étant adaptée en fonction de la présence d'une proline ou d'une sérine sur ladite position, la posologie du produit pharmaceutique étant adaptée en présence d'un ou plusieurs desdits variants génomiques ; ou
c) pour savoir si la quantité de l'ARNm et/ou de la protéine CLEC1B présent dans ledit échantillon est ou non différente de celle d'un ou plusieurs échantillons de référence prélevés, ladite posologie étant adaptée selon que la quantité de protéine et/ou d'ARNm dans l'échantillon prélevé de l'individu est ou non différente de celle de l'échantillon de référence ou des échantillons de référence.

8. Procédé pour identifier les individus répondant à un produit pharmaceutique destiné au traitement et/ou à la prévention de troubles cardiovasculaires et/ou thrombotiques, dans lequel on analyse le SNP sur la position 251 du gène CLEC1B selon SEQ ID N° 1.

9. Procédé pour identifier les individus répondant à un produit pharmaceutique destiné au traitement et/ou à la prévention de troubles cardiovasculaires et/ou thrombotiques, par analyse d'un acide nucléique de CLEC1B pour ce qui est de la présence d'un polymorphisme mononucléotidique thymidine (T) → cytidine (C) sur la position 251 de la séquence d'acide nucléique de CLEC1B selon la séquence de référence SEQ ID N° 1 et/ou par analyse d'une protéine CLEC1B pour ce qui est de la présence des polymorphismes protéiques sérine (Ser) → proline (Pro) sur la position 24 de la protéine CLEC1B selon la séquence de référence SEQ ID N° 2.

10. Procédé selon l'une quelconque des revendications précédentes, dans lequel les troubles cardiovasculaires et/ou thrombotiques sont l'angine de poitrine, l'angine de poitrine instable, l'infarctus du myocarde, l'infarctus précoce du myocarde, les troubles vasculaires périphériques, la maladie coronarienne, l'hypertension, l'accident vasculaire cérébral, le déficit neurologique ischémique prolongé réversible (PRIND), l'accident ischémique transitoire (TIA), ou un trouble nécessitant la mise en oeuvre d'une angioplastie coronarienne.

11. Procédé selon l'une quelconque des revendications précédentes, comprenant l'analyse d'un échantillon prélevé d'un individu, l'individu dont on examine l'échantillon prélevé ayant un trouble du métabolisme du glucose, et souffrant de préférence de diabète et d'une manière particulièrement préférée de diabète de type I.

12. Procédé selon l'une quelconque des revendications précédentes, dans lequel l'individu dont on examine l'échantillon prélevé souffre de troubles cardiovasculaires et/ou thrombotiques.

13. Procédé selon l'une quelconque des revendications précédentes, dans lequel l'échantillon est un échantillon de mammifère et de préférence un échantillon humain.

14. Procédé selon l'une quelconque des revendications précédentes, dans lequel l'échantillon est un échantillon histologique, un échantillon biopsique, un extrait cellulaire, une ou plusieurs cellules, ou un fluide corporel prélevé.

15. Procédé selon l'une quelconque des revendications précédentes 1 à 9, dans lequel le CLEC1B est utilisé sous forme d'une molécule isolée.

16. Procédé selon l'une des revendications 16 ou 17, dans lequel les SNP sont : une cytidine sur la position 251 de la séquence de CLEC1B sur l'un des allèles, ou les deux, du gène CLEC1B (selon SEQ ID N° 2).

17. Procédé selon les revendications 1 à 4 ou 6, dans lequel l'identification des polymorphismes protéiques suivants indique la maladie ou un risque accru : une proline sur la position 24 de la protéine CLEC1B selon SEQ ID N° 2.

18. Procédé selon l'une quelconque des revendications 1 à 3 ou 6 à 10, dans lequel le type de nucléotide sur la position 251 du gène CLEC1B selon SEQ ID N° 1 est déterminé au moyen d'une ou plusieurs amorces ou sondes appropriées.

19. Procédé selon la revendication 20, dans lequel le type de nucléotide est identifié par PCR, transfert de Southern, hybridation sur microréseau ou puce.

20. Procédé selon la revendication 7, dans lequel le changement de quantité de l'ARNm est analysé de préférence par utilisation d'une ou plusieurs amorces appropriées à l'amplification de l'ADNc de CLEC1B, ou d'une ou plusieurs sondes convenant à une hybridation avec l'ADNc ou l'ARNm de CLEC1B, dans les conditions standard.

21. Procédé selon l'une des revendications 20 à 23, **caractérisé par** un ensemble d'amorces contenant une amorce, ou les deux amorces, de la séquence telle que définie dans SEQ ID N° 4 et 5, ou une sonde telle que définie dans la séquence SEQ ID N° 3.

22. Amorce d'acide nucléique, ayant une séquence d'acide nucléique selon SEQ ID N° 4 ou 5.

23. Sonde d'acide nucléique ayant la séquence d'acide nucléique selon SEQ ID N° 3.


### SEQUENCE LISTING

<110> Sanofi-Aventis
<120> USE OF CLEC1B FOR THE DETERMINATION OF CARDIOVASCULAR AND THROMBOTIC RISK
<130> DE2007/042
<160> 5
<170> PatentIn version 3.3
<210> 1
   <211> 963 <212> DNA
   <213> Homo sapiens
<400> 1
   ctatgaagaa gcttcctgga aaacaataag caaaggaaaa caaatgtgtc ccatctcaca    60
   tggttctacc ctactaaaga caggaagatc ataaactgac agatactgaa attgtaagag   120
   ttggaaacta cattttgcaa agtcattgaa ctctgagctc agttgcagta ctcgggaagc   180
   catgcaggat gaagatggat acatcacctt aaatattaaa actcggaaac cagctctcgt   240
   ctccgttggc cctgcatcct cctcctggtg gcgtgtgatg gctttgattc tgctgatcct   300
   gtgcgtgggg atggttgtcg ggctggtggc tctggggatt tggtctgtca tgcagcgcaa   360
   ttacctacaa gatgagaatg aaaatcgcac aggaactctg caacaattag caaagcgctt   420
   ctgtcaatat gtggtaaaac aatcagaact aaagggcact ttcaaaggtc ataaatgcag   480
   cccctgtgac acaaactgga gatattatgg agatagctgc tatgggttct tcaggcacaa   540
   cttaacatgg gaagagagta agcagtactg cactgacatg aatgctactc tcctgaagat   600
   tgacaaccgg aacattgtgg agtacatcaa agccaggact catttaattc gttgggtcgg   660
   attatctcgc cagaagtcga atgaggtctg gaagtgggag gatggctcgg ttatctcaga   720
   aaatatgttt gagtttttgg aagatggaaa aggaaatatg aattgtgctt attttcataa   780
   tgggaaaatg caccctacct tctgtgagaa caaacattat ttaatgtgtg agaggaaggc   840
   tggcatgacc aaggtggacc aactacctta atgcaaagag gtggacagga taacacagat   900
   aagggcttta ttgtacaata aaagatatgt atgaatgcat cagtagctga aaaaaaaaaa   960
   aaa                                                                 963
<210> 2
   <211> 229
   <212> PRT
   <213> Homo sapiens
<400> 2
   Met Gln Asp Glu Asp Gly Tyr Ile Thr Leu Asn Ile Lys Thr Arg Lys
   1               5                   10                  15
   Pro Ala Leu Val Ser Val Gly Pro Ala Ser Ser Ser Trp Trp Arg Val
               20                  25                  30
   Met Ala Leu Ile Leu Leu Ile Leu Cys Val Gly Met Val Val Gly Leu
   35 40 45
   Val Ala Leu Gly Ile Trp Ser Val Met Gln Arg Asn Tyr Leu Gln Asp
   50 55 60
   Glu Asn Glu Asn Arg Thr Gly Thr Leu Gln Gln Leu Ala Lys Arg Phe
   65 70 75 80
   Cys Gln Tyr Val Val Lys Gln Ser Glu Leu Lys Gly Thr Phe Lys Gly
   85 90 95
   His Lys Cys Ser Pro Cys Asp Thr Asn Trp Arg Tyr Tyr Gly Asp Ser
   100 105 110
   Cys Tyr Gly Phe Phe Arg His Asn Leu Thr Trp Glu Glu Ser Lys Gln
   115 120 125
   Tyr Cys Thr Asp Met Asn Ala Thr Leu Leu Lys Ile Asp Asn Arg Asn
   130 135 140
   Ile Val Glu Tyr Ile Lys Ala Arg Thr His Leu Ile Arg Trp Val Gly
   145 150 155 160
   Leu Ser Arg Gln Lys Ser Asn Glu Val Trp Lys Trp Glu Asp Gly Ser
   165 170 175
   Val Ile Ser Glu Asn Met Phe Glu Phe Leu Glu Asp Gly Lys Gly Asn
   180 185 190
   Met Asn Cys Ala Tyr Phe His Asn Gly Lys Met His Pro Thr Phe Cys
   195 200 205
   Glu Asn Lys His Tyr Leu Met Cys Glu Arg Lys Ala Gly Met Thr Lys
   210 215 220
   Val Asp Gln Leu Pro
   225
<210> 3
   <211> 401
   <212> DNA
   <213> Homo sapiens
<400> 3
   agaatggatg tatggtatag gggtgggcca tgcctattgt tgatgatcat gaggagaaca      60
   caggtgagat ctgccagcaa agctctttct attacttgga ggaccctgag aaggcagggg     120
   caggggcagg ggtgagcctt ccccatagct aacccatact gcctgctcct tgatgtcttt     180
   attatatgcc tacagttggc yctgcatcct cctcctggtg gcgtgtgatg gctttgattc     240
   tgctgatcct gtgcgtgggg atggttgtcg ggctggtggc tctggggatt tggtgtaagt     300
   gttgactctg ccagaaattt gactggagga aggtaatact gaagggtcat ggcatatccc     360
   actaagcttc tcaatggtcg ctatttgtct gtttatcact t                         401
<210> 4
   <211> 24
   <212> DNA
   <213> Artificial
<220>
   <223> Forward Primer
<400> 4
   tgcctgctcc ttgatgtctt tatt 24
<210> 5
   <211> 23
   <212> DNA
   <213> Artificial
<220>
   <223> Reverse Primer
<400> 5
   tcagcagaat caaagccatc aca 23